(19) Europäisches Patentamt
European Patent Office
Office européen des brevets

(11) Numéro de publication: **0 159 254**
**B1**

(12) # FASCICULE DE BREVET EUROPEEN

(45) Date de publication du fascicule du brevet: **07.10.87**

(21) Numéro de dépôt: **85400610.3**

(22) Date de dépôt: **28.03.85**

(51) Int. Cl.⁴: **C 07 C 149/23**, C 07 C 153/09, A 61 K 31/00, C 07 D 211/26, C 07 D 211/58

(54) **Nouveaux dérivés de l'w-mercaptopropanamide et leurs sels, leur préparation, leur application comme médicaments et les compositions les renfermant.**

(30) Priorité: **29.03.84 FR 8404917**

(43) Date de publication de la demande: **23.10.85 Bulletin 85/43**

(45) Mention de la délivrance du brevet: **07.10.87 Bulletin 87/41**

(84) Etats contractants désignés: **AT BE CH DE FR GB IT LI LU NL SE**

(56) Documents cités:
**EP - A - 0 001 989**
**EP - A - 0 035 868**
**GB - A - 2 076 809**
**GB - A - 2 077 719**
**US - A - 4 148 815**

(73) Titulaire: **ROUSSEL-UCLAF, 35, boulevard des Invalides, F-75007 Paris (FR)**

(72) Inventeur: **Delevallée, Françoise, 48-50, avenue de la Dame Blanche, F-94120 Fontenay-sous-Bois (FR)**
Inventeur: **Vevert, Jean-Paul, 55, rue Rouget de L'Isle, F-93500-Pantin (FR)**

(74) Mandataire: **Bourgouin, André et al, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

La présente invention concerne de nopuveaux dérivés de l'ω-mercapto propanamide et de ses homologues, leur procédé de préparation, leur application comme médicaments et les compositions les renfermant.

Le brevet européen 1989 décrit des dérivés de mercaptopropanamide et notamment des dérivés portant une fonction carbonyle reliée à la fonction amide.

Ces produits sont présentés comme des immunorégulateurs utiles dans de très nombreuses maladies mais ne sont pas décrits comme analgésiques.

Le brevet anglais 2 076 809 décrit des inhibiteurs de collagénase utiles dans le traitement de l'arthrite rhumatoïde dont le substituant porté par la fonction amide est différent de celui des produits de la présente demande.

Le brevet US 4 148 815 décrit un procédé d'extraction de métaux utilisant le N-phényl 2-mercaptoacétamide et ses homologues.

L'invention a pour objet les composés de formule (I):

$$R_1S-(CH_2)_n-\underset{\underset{R_2}{|}}{CH}-\underset{\underset{O}{||}}{C}-NH-(CH_2)_m-R_3 \qquad (I)$$

dans laquelle $R_1$ représente un atome d'hydrogène ou un radical $-\underset{\underset{O}{||}}{C}-R'_1$, $R'_1$ étant un radical alcoyle renferment de 1 à 5 atomes de carbone ou un radical aryle éventuellement substitué par un radical hydroxy, un radical alcoyle renfermant de 1 à 5 atomes de carbone, un radical alcoxy renfermant de 1 à 5 atomes de carbone, le radical nitro ou un atome d'halogène, n représente un nombre entier pouvant varier de 1 à 5, $R_2$ représente un atome d'hydrogène, un radical alcoyle renfermant de 1 à 5 atomes de carbone, un radical aryle ou arylalcoyle renfermant de 6 à 15 atomes de carbone éventuellement substitué par un radical alcoyle renfermant de 1 à 5 atomes de carbone, un radical alcoxy renfermant de 1 à 5 atomes de carbone, un radical hydroxyle, un atome d'halogène ou un radical trifluorométhyle, m représente un nombre entier pouvant varier de 0 à 5, $R_3$ représente:

— soit un radical cyclique saturé renfermant de 3 à 12 atomes de carbone, lequel pouvant être accolé à un radical phényle, $R_3$ ne pouvant toutefois représenter un radical cyclohexyle lorsque $R_2$ et $R_1$ représentent à la fois un atome d'hydrogène et que $n = 1$ et $m = 0$;

— soit un radical phényle éventuellement substitué par un ou plusieurs radicaux choisis parmi les radicaux alcoyles renfermant de 1 à 5 atomes de carbone, alcoxy renfermant de 1 à 5 atomes de carbone, hydroxyle, nitro, halogène, trifluorométhyle et $-N\!\begin{smallmatrix}X\\X'\end{smallmatrix}$, X et X' représentant un atome d'hydrogène ou un radical alcoyle renfermant de 1 à 5 atomes de carbone, ou un radical phényle auquel est accolé un radical cyclique saturé renfermant de 5 à 9 atomes de carbone;

— soit un radical hétérocyclique choisi parmi les radicaux thiazolyle, 4-5-dihydrothiazolyle, pyridinyle, oxazolyle, isoxazolyle, imidazolyle, pyrimidyle, tétrazolyle, benzimidazolyle, benzothiazolyle, benzoxazolyle, éventuellement substitués par un radical alcoyle renfermant de 1 à 5 atomes de carbone, ou pipéridinyle substitué par un radical aralcoyle renfermant de 7 à 15 atomes de carbone, à la condition que lorsque $R_3$ représente un radical phényle éventuellement substitué par au moins un des radicaux cités précédemment ou lorsque $R_3$ représente un des radicaux hétérocycliques mentionnés ci-dessus, éventuellement substitués par un radical alcoyle renfermant de 1 à 5 atomes de carbone, m ne peut représenter la valeur 0 et à la condition que:

a) lorsque $n = 1$ ou 2, $m = 1$, 2 ou 3 et $R_1 = R_2 = H$, $R_3$ ne soit pas un radical phényle éventuellement substitué par un ou deux radicaux alcoyles renfermant de 1 à 5 atomes de carbone;

b) lorsque $m = n = 1$ et $R_1 = CH_3-\underset{\underset{O}{||}}{C}-$ et $R_2 = CH_3$ $R_3$ ne soit pas un radical phényle, ainsi que leurs sels d'addition avec les acides.

Lorsque $R_1$ représente un radical $-\underset{\underset{O}{||}}{C}-R'_1$, $R'_1$ est de préférence un radical méthyle ou éthyle.

Lorsque $R'_1$ est un radical aryle, il s'agit de préférence d'un radical phényle.

Lorsque $R'_1$ est un radical aryle substitué, il s'agit de préférence d'un radical aryle substitué par un radical choisi dans le groupe formé par le radical hydroxy, les radicaux méthyle et éthyle, les radicaux méthoxy et éthoxy, le radical nitro et l'atome de chlore.

n est de préférence égal à 1 ou à 2.

Lorsque $R_2$ représente un radical alcoyle, il s'agit de préférence du radical méthyle, éthyle, n-propyle, n-butyle, n-pentyle, isopropyle ou isobutyle.

Lorsque $R_2$ est un radical aryle, il s'agit de préférence d'un radical phényle.

Lorsque $R_2$ est un radical arylalcoyle, il s'agit de préférence d'un radical benzyle ou phénéthyle.

Lorsque $R_2$ est un radical aryle ou arylalcoyle substitué, il s'agit de préférence d'un radical aryle ou arylalcoyle substitué, par un radical méthyle ou éthyle, un radical méthoxy ou éthoxy, un radical hydroxyle, un atome de chlore ou un radical trifluorométhyle.

m est de préférence égal à 0, 1, 2 ou 3.

Lorsque $R_3$ est un radical cyclique saturé, il s'agit de préférence d'un radical cyclopropane, cyclobutane, cyclopentane, cyclohexane, cycloheptane, cyclooctane, cyclononane, cyclodécane, cycloundécane ou cyclododécane.

Lorsque $R_3$ est un radical cyclique saturé auquel est accolé un radical phényle, le radical ainsi formé est de préférence le radical 1,2,3,4-tetrahydro-1-naphthyl ou 1,2,3,4-tétrahydro 2-naphthyl ou 2,3-dihydro 1H-inden-1-yl ou 2,3-dihydro 1H-inden-2-yl.

Lorsque $R_3$ représente un radical phényle substitué, il s'agit de préférence d'un radical phényle substitué par un ou plusieurs radicaux choisis dans le groupe formé par le radical hydroxy, les radicaux méthyle et éthyle, les radicaux méthoxy et éthoxy, le radical trifluorométhyle, le radical nitro, l'atome de chlore, les radicaux amino et diméthylamino.

Lorsque $R_3$ représente un radical phényle auquel est accolé un radical cyclique saturé, le radical ainsi formé est de préférence le radical 5,6,7-tétrahydro 1-naphthyl ou 5,6,7,8-tétrahydro 2-naphtyl ou 2,3-dihydro 1H-indén-4-yl ou 2,3-dihydro 1H-indén-5-yl.

Lorsque $R_3$ représente un radical hétérocyclique substitué par un radical alcoyle, il s'agit de préférence d'un radical hétérocyclique substitué par un radical méthyle ou éthyle.

Lorsque $R_3$ représente un radical pipéridinyle substitué par un radical aralcoyle, on entend de préférence par aralcoyle un radical benzyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane et éthane sulfoniques, arylsulfoniques, tels que les acides benzène et paratoluène sulfoniques et arylcarboxyliques.

L'invention a notamment pour objet les composés de formule (I), caractérisés en ce que $R_1$ est atome d'hydrogène et ceux caractérisés en ce que $R_1$ est un radical acétyle.

L'invention a particulièrement pour objet les composés de formule (I), caractérisés en ce que $n = 1$ et $R_2$ est un radical benzyle ou un atome d'hydrogène et ceux caractérisés en ce que $m = 0$, 1 ou 2 et $R_3$ représente un radical cyclique saturé de 3 à 8 atomes de carbone éventuellement accolé à un radical phényle ou $R_3$ représente un radical phényle auquel est accolé un radical cyclique saturé renfermant de 5 à 9 atomes de carbone.

L'invention concerne aussi tout particulièrement les composés de formule (I), caractérisés en ce que $m = 1$ ou 2 et $R_3$ représente un radical phényle ou un radical pyridinyle.

Parmi les composés de l'invention, on peut citer les produits décrits dans les exemples et tout particulièrement:

- le N-cyclohexyl α-(mercapto méthyl) benzène propanamide,
- l'α-(mercaptométhyl) N-(2-phényl éthyl) benzène propanamide,
- le N-(2,3-dihydro 1H-inden-2-yl) 3-mercapto propanamide,
- l'α-(mercaptométhyl) N-(2-pyridinylméthyl) benzène propanamide,
- l'α-(mercaptométhyl) N-(3-pyridinylméthyl) benzène propanamide,
- l'α-(mercaptométhyl) N-(4-pyridinylméthyl) benzène propanamide, ainsi que leurs sels d'addition avec les acides.

L'invention a également pour objet un procédé de préparation des produits de formule (I), dans laquelle $R_1$, $R_2$, $R_3$ et m ont les significations données précédemment et dans laquelle $n = 1$, caractérisé en ce que l'on soumet un acide de formule (II):

$$R'_1-\overset{\overset{\displaystyle O}{\|}}{C}-S-CH_2-\underset{\underset{\displaystyle R_2}{|}}{CH}-COOH \qquad (II)$$

dans laquelle $R'_1$ et $R_2$ ont les significations déjà indiquées, ou un dérivé fonctionnel de cet acide, à l'action d'un produit de formule (III):

$$H_2N-(CH_2)_m-R_3 \qquad (III)$$

dans laquelle $R_3$ et m ont les significations déjà indiquées, pour obtenir un produit de formule (I), dans laquelle $R_1 = R'_1-\underset{\underset{\displaystyle O}{\|}}{C}-$, $R'_1$ ainsi que $R_2$, $R_3$ et m ayant les significations déjà indiquées et dans laquelle $n = 1$, produits de formule (I) que l'on saponifie, le cas échéant, pour obtenir un produit de formule (I) dans laquelle $R_1$ représente un atome d'hydrogène et produits de formule (I) que l'on salifie le cas échéant, par action d'un acide.

L'invention concerne aussi un procédé de préparation des produits de formule (I), dans laquelle $R_1$, $R_2$, $R_3$ et m ont les significations données précédemment et dans laquelle n représente un nombre entier pouvant varier de 1 à 5, caractérisé en ce que l'on soumet un acide de formule (IV):

$$X-(CH_2)_n-\overset{\overset{\displaystyle R_2}{|}}{CH}-COOH \qquad (IV)$$

dans laquelle X est un atome d'halogène, et dans laquelle n et $R_2$ ont les significations données ci-dessus, ou un dérivé fonctionnel de celui-ci, à l'action d'un produit de formule (III) $H_2N(CH_2)_mR_3$ précédent, pour obtenir un produit de formule (V):

$$X-(CH_2)_n-\overset{\overset{\displaystyle R_2}{|}}{CH}-COHN-(CH_2)_m-R_3 \qquad (V)$$

dans laquelle X, n, m, $R_2$ et $R_3$ ont les significations précédentes, que l'on soumet à l'action de l'anion d'un thioacide de formule (VI):

$$R'_1-\underset{\underset{\displaystyle O}{\|}}{C}-SH \qquad (VI)$$

dans laquelle $R'_1$ a la signification précédente pour obtenir un produit de formule (I) dans laquelle $R_1 = R'_1-\underset{\underset{\displaystyle O}{\|}}{C}-$, $R'_1$, $R_2$, $R_3$, n et m ayant les significations données ci-dessus, produit de formule (I) que l'on saponifie, le cas échéant, pour obtenir un produit de formule (I) dans laquelle $R_1$ représente un atome d'hydrogène et produits de formule (I)

que l'on salifie, le cas échéant, par action d'un acide.

Dans les conditions préférentielles de mise en œuvre de l'invention 40 les procédés ci-dessus décrits sont réalisés de la manière suivante:

— les dérivés fonctionnels des acides de formule (II) et (IV) sont des chlorures d'acides;

— dans le produit de formule (IV), X est un atome de chlore;

— les réactions de condensation entre les produits de formule (II) et (IV) et les produits de formule (III) s'effectuent au sein d'un solvant tel que l'éther éthylique, le tétrahydrofuranne, le diméthylformamide ou d'un solvant chloré, notamment le chlorure de méthylène mais d'autres solvants tels le 1,1-dichloroéthane, le chloroforme, le tétrachlorure de carbone peuvent aussi être utilisés;

— la réaction de condensation entre les produits de formule (II) et les produits de formule (III) s'effectue à une température se situant entre −10°C et 0°C;

— la saponification des produits de formule (I) dans laquelle $R_1 = R'_1$-C- s'effectue par des moyens
||
O
usuels, notamment en utilisant un hydroxyde de métal alcalin.

Les composés de formule (I) tels que définis ci-dessus ainsi que leurs sels sont doués d'intéressantes propriétés pharmacologiques. Ils sont doués, en particulier, de très intéressantes propriétés inhibitrices de l'enképhalinase et sont doués d'une très bonne activité analgésique.

L'enképhalinase est une dipeptidylcarboxypeptidase qui hydrolyse spécifiquement la méthionine et la leucine enképhaline entre le 3ème et le 4ème acide aminé, libérant ainsi un tripeptide Tyr-Gly-Gly (Swerts, J.P., Perdrisot, R., Patey, G., DeLa Baume, S., and Schwartz, J.C., Europ. J. Pharmacol., 1979, 57, 279).

L'enképhalinase participe ainsi directement à la dégradation physiologique des enképhalines, ligands naturels endogènes des récepteurs opiacés. Les composés de l'invention, qui retardent la dégradation des enképhalines, stimulent donc les réactions de défense de l'organisme contre la douleur.

Ces propriétés justifient leur application en thérapeutique et l'invention a également pour objet, à titre de médicaments, les produits tels que définis par la formule (I) ci-dessus, ainsi que leurs sels d'addition avec les acides pharmaceutiquement acceptables.

La présente invention a tout particulièrement pour objet, à titre de médicaments:

— le N-cyclohexyl α-(mercapto méthyl) benzène propanamide,
— l'α-(mercapto méthyl) N-(2-phényl éthyl) benzène propanamide,
— le N-(2,3-dihydro 1H-inden-2-yl) 3-mercapto propanamide,
— l'α-(mercaptométhyl) N-(2-pyridinylméthyl) benzène propanamide,
— l'α-(mercaptométhyl) N-(3-pyridinylméthyl) benzène propanamide,
— l'α-(mercaptométhyl) N-(4-pyridinylméthyl) benzène propanamide,

ainsi que leurs sels d'addition avec les acides pharmaceutiquement acceptables.

Les médicaments, objet de l'invention, peuvent être utilisés dans le traitement des algies musculaires, articulaires ou nerveuses, des douleurs dentaires, des zonas et des migraines, ainsi que dans le traitement des affections rhumatismales et aussi à titre de traitement complémentaire dans les états infectieux et fébriles.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes. les gels et les préparations en aérosols: elles sont préparées slon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif, par jour, par voie orale.

Certains des composés de formule (II) et (IV) utilisés comme produits de départ du procédé de l'invention sont décrits et préparés dans le brevet ONDETTI et al. US 4 053 651.

Les produits de formule (II) et (IV) non décrits dans ce brevet peuvent être préparés comme indiqué dans ce brevet.

Les exemples suivants illustrent l'invention sans toutefois la limiter.

*Exemple 1*

*α-[(acétylthio)méthyl] N-cyclohexyl benzène propanamide.*

*Stade A:* Chlorure d'α-(acétylthiométhyl) benzène propanoyle.

On dissout 1,21 g d'acide α-benzyl acrylique (produit décrit et préparé selon C. Mannich et Coll. Chem. Ber, 57B, 1116-8, (1924) dans 0,8 g d'acide thioacétique, garde une heure à température ambiante sous argon, chauffe une heure à 100°C, élimine l'excès d'acide thioacétique sous pression réduite. A l'huile résiduelle, on ajoute sous agitation sous argon et à 0°C, goutte à goutte, 1,8 g de chlorure de thionyle. On garde une nuit à température ambiante, élimine l'excès de chlorure en thionyle sous pression réduite, ajoute 30 $cm^3$ de benzène que l'on élimine ensuite sous presssion réduite. Après distillation

de l'huile résiduelle, on obtient 1,87 g de produit attendu.

Eb. = 150°C ($10^{-2}$ Torr).

*Stade B:* α-[(acétylthio)méthyl] N-cyclohexyl-benzène propanamide.

On ajoute à -5°C, -10°C, sous argon, en 1 heure 6,42 g de produit préparé au stade A précédent dans 4,96 g d'une solution de cyclohexyl amine dissout dans 100 $cm^3$ de chlorure de méthylène. On élimine les solvants sous pression réduite à 30°C, reprend le résidu dans 300 $cm^3$ d'éther, filtre, lave la phase organique avec de l'acide chlorhydrique 0,1N, du carbonate acide de sodium, de l'eau et avec une solution de chlorure de sodium saturée. On sèche la phase organique, filtre, concentre à sec le filtrat sous pression réduite. On obtient une première fraction de produit attendu de 3,8 g. On reprend le chlorhydrate de cyclohexylamine par 200 $cm^3$ de chlorure de méthylène, lave avec de l'acide chlorhydrique 0,1N, sèche la phase organique, essore, concentre sous pression réduite. On obtient une deuxième fraction de 3,7 g de produit attendu. On réunit les 2 fractions obtenues dans 150 $cm^3$ de chlorure de méthylène, filtre, élimine les solvants et sèche sous pression réduite. On obtient 7,5 g de produit attendu qui présente un Rf de 0,35 en chromatographie sur silice en éluant avec un mélange cyclohexane-acétate d'éthyle (75-25).

*Exemple 2*

*N-cyclohexyl α-(mercaptométhyl) benzène propanamide.*

On introduit en 1 heure à -10°C sous argon, 235,7 $cm^3$ de soude 0,1N dans 7,5 g de produit obtenu à l'exemple 1 précédent en solution dans 250 $cm^3$ de méthanol. On laisse revenir lentement à 0°C et ajoute à cette température 235,7 $cm^3$ d'acide chlorhydrique 0,1N en 30 minutes jusqu'à pH 6-7. On élimine le méthanol sous pression réduite puis extrait au chlorure de méthylène. On sèche la phase organique, concentre à sec sous pression réduite. On obtient 6,5 g de produit brut que l'on chromatographie sur silice en éluant avec un mélange cyclohexane-chlorure de méthylène (1-9). On obtient 3,1 g de produit attendu fondant à 117°C.

*Analyse:* $C_{16}H_{23}NOS$: PM 277,431

| | | | | |
|---|---|---|---|---|
| Calculé: | C% 69,3 | H% 8,4 | N% 5,1 | S% 11,6 |
| Trouvé: | 69,4 | 8,5 | 4,9 | 11,6 |

*Exemple 3*

*α-[(acétylthio) méthyl] N-cyclohexyl propanamide.*

*Stade A:* Chlorure de l'acide 3-acétylthio 2-méthyl propanoïque.

On introduit 261 g d'acide 3-acétylthio 2-méthyl propanoïque [préparé selon Skeldon N. Lewis dans J. Het. Chem. (4) 571 (1971)], dans 520 $cm^3$ de chlorure du thionyle. On chauffe la solution progressivement, en 3 heures, jusqu'à obtention du reflux et le maintient pendant 2 heures; il se produit un intense dégagement gazeux. Après distillation du chlorure de thionyle, on obtient une huile que l'on rectifie sous un vide de 15 mm de Hg à 106°C. On obtient ainsi 182 g de produit attendu utilisé tel que pour le stade suivant.

*Stade B:*

α-[(acétylthio)méthyl] N-cyclohexyl propanamide.

On introduit en 30 minutes, sous argon et sous agitation, 2 g de produit préparé au stade A, dans 2,20 g de cyclohexylamine en solution dans 100 $cm^3$ de chlorure de méthylène refroidit à -9°C.

On maintient l'agitation 2 heures à -5°C, puis 1 heure à température ambiante et élimine le chlorure de méthylène sous pression réduite. On reprend le résidu par 300 $cm^3$ d'éther et agite 15 minutes, filtre le chlorhydrate de cyclohexylamine, lave le filtrat par de l'acide chlorhydrique N, de l'eau, du carbonate acide de sodium en solution saturée, puis avec de l'eau. On sèche la phase organique, filtre, concentre sous pression réduite. On empâte le résidu dans 100 $cm^3$ de pentane, essore, lave au pentane, sèche sous pression réduite et obtient 2,102 g de produit fondant à 82-83°C.

*Exemple 4*

*N-cyclohexyl 3-mercapto 2-méthyl propanamide.*

On introduit à -5°C, sous argon et sous agitation en 30 minutes, 85 $cm^3$ de soude 0,1N dans 2,066 g de produit obtenu à l'exemple 3 en solution dans 125 $cm^3$ de méthanol.

On maintient l'agitation 1 heure à -5°C, acidifie à 0°C à pH 5-6 avec 85 $cm^3$ d'acide chlorhydrique 0,1N, élimine le méthanol sous pression réduite, essore, lave à l'eau, sèche sous pression réduite et obtient un résidu que l'on chromatographie sur silice en éluant avec du chloroforme. On obtient 1,351 g de produit attendu fondant à 115°C.

*Analyse:* $C_{10}H_{19}NOS$ PM: 201,333

| | | | | |
|---|---|---|---|---|
| Calculé: | C% 59,66 | H% 9,51 | N% 6,96 | S% 15,92 |
| Trouvé: | 59,7 | 9,6 | 6,9 | 15,8 |

*Exemple 5*

*α-[(acétylthio)méthyl] N-(phényl méthyl) benzène propanamide.*

On introduit en 30 minutes, à -5°C environ, 6,42 g de chlorure d'α-(acétylthio méthyl) benzène propanoyle en solution dans 250 $cm^3$ de chlorure de méthylène et préparé comme indiqué au stade A de l'exemple 1, dans 5,36 g de benzyl amine dissoute dans 250 $cm^3$ de chlorure de méthylène.

On laisse à température ambiante pendant 16 heures, élimine les solvants sous pression réduite et précipite le chlorhydrate de benzylamine par addition de 400 $cm^3$ d'éther. On filtre le précipité formé, lave le filtrat à l'acide chlorhydrique 0,1N et à l'eau jusqu'à pH = 6, sèche, élimine les solvants. On chromatographie sur silice le résidu obtenu en éluant avec un mélange chloroforme-acétate d'éthyle (98-2). On obtient 8,8 g de produit attendu fondant à 90°C.

*Exemple 6*

*α-(mercapto méthyl) N-(phényl méthyl) benzène propanamide.*

On introduit en 1 heure, sous argon, sous agitation, vers 0°C, -5°C, 269 $cm^3$ de soude 0,1N dans 8,8 g de produit obtenu à l'exemple 5 en solution dans 250 $cm^3$ de méthanol. On laisse revenir à 0°C en 1 heure, ajoute 269 $cm^3$ d'acide chlorhydrique 0,1N en 1 heure, élimine le méthanol sous pression réduite, extrait la phase aqueuse au chlorure de méthylène, sèche, filtre et concentre à sec sous pression réduite. On obtient 6,95 g de résidu sous forme d'une huile que l'on chromatographie sur silice en éluant avec un mélange chlorure de méthylène-cyclohexane (8-2). On obtient 3,5 g de produit attendu fondant à 70°C.

*Analyse:* $C_{17}H_{19}NOS$ PM: 285,411

| | | | | |
|---|---|---|---|---|
| Calculé: | C% 71,5 | H% 6,7 | N% 4,9 | S% 11,2 |
| Trouvé: | 71,7 | 6,9 | 4,8 | 11,2 |

*Exemple 7*

*α-[(acétylthio)méthyl] N-(2-phényl éthyl) benzène propanamide.*

Dans 6,059 g de phényl éthylamine en solution dans 250 $cm^3$ de chlorure de méthylène, on introduit en 30 minutes, à 0°C, sous agitation et sous argon, 6, 419 g de chlorure d'α-(acétylthio méthyl) benzène propanoyle préparé comme indiqué au stade A de l'exemple 1 en solution dans 250 $cm^3$ de chlorure de méthylène et agite pendant 16 heures en laissant remonter la température à 20°C. On filtre le chlorhydrate de phényl éthylamine formé, lave le filtrat par de l'acide chlorhydrique 1N, de l'eau, du carbonate acide de sodium en solution saturée, puis à l'eau jusqu'à pH = 6-7. On sèche la phase organique, filtre, concentre à sec sous pression réduite. On empâte le résidu obtenu dans 50 $cm^3$ d'éther, essore, lave à l'éther, sèche sous pression réduite et obtient 5,476 g de produit obtenu fondant à 64°C.

*Exemple 8*

*α-(mercapto méthyl) N-(2-phényl éthyl) benzène propanamide.*

On opère comme indiqué à l'exemple 4 à partir de 160 $cm^3$ de soude 0,1N, de 5,442 g de produit obtenu à l'exemple 7 en solution dans 325 $cm^3$ de méthanol et obtient 3,529 g de produit attendu fondant à 70°C.

*Analyse:* $C_{18}H_{21}NOS$ PM: 299,438

| | | | | |
|---|---|---|---|---|
| Calculé: | C% 72,20 | H% 7,07 | N% 4,68 | S% 10,7 |
| Trouvé: | 72,5 | 7,2 | 4,7 | 10,6 |

*Exemple 9*

*3-(acétylthio) N-(2-phényl éthyl) propanamide.*

*Stade A:* Chlorure d'acide 3-(acétylthio) propanoïque.

On introduit en 30 minutes sous agitation et sous argon, 100 $cm^3$ d'acide thioacétique, dans 75 $cm^3$ d'acide acrylique, en maintenant la température entre 22°C et 35°C. On agite une nuit à température ambiante puis 1 heure 30 à 100°C. En ramenant à la température ambiante, la masse recristallise. On élimine l'excès d'acide thioacétique sous pression réduite et obtient 170 g d'acide 3-(acétylthio) propanoïque (F = 40-45°C). On le dissout dans 170 $cm^3$ d'éther éthylique anhydride et ajoute sous agitation et sous argon 130 $cm^3$ de chlorure de thionyle. On agite une nuit à température ambiante, elimine l'excès de chlorure de thionyle sous pression réduite puis par entraînement au benzène.

Après distillation de l'huile résiduelle, on obtient 146,9 g de produit attendu (Eb. 0,5 mm/Hg = 63-64°C).

*Stade B:* 3(acétylthio) N-(2-phényl éthyl) propanamide.

On procède comme indiqué à l'exemple 5 à partir de 2,314 g de chlorure d'acide 3-acétylthio propanoïque en solution dans 150 $cm^3$ de chlorure de méthylène et préparé comme indiqué au stade A précédent et de 3,366 g de phényl éthylamine en solution dans 150 $cm^3$ de chlorure de méthylène. On obtient 2,177 g de produit attendu fondant à 69-70°C après chromatographie sur silice en éluant par un mélange chlorure de méthylène-acétate d'éthyle (95-5).

*Example 10*

*3-acétylthio N-(2,3-dihydro 1H-inden-2-yl) propanamide.*

On opère comme indiqué à l'exemple 3 à partir d'une solution de 3,036 g de chlorhydrate de 2-amino indane et de 45 mmoles de triéthylamine dans 150 $cm^3$ de chlorure de méthylène et de 2,500 g de chlorure d'acide 3-(acétylthio) propanoïque préparé comme indiqué au stade A de l'exemple 9.

On obtient 3,88 g de produit attendu fondant à 74°C.

*Exemple 11*

*N-(2,3-dihydro 1H-inden-2-yl) 3-mercapto propanamide.*

On ajoute 150 $cm^3$ de soude 0,1N sous argon et sous agitation entre -7°C et -10°C, en 30 minutes, à 3,87 g de produit obtenu à l'exemple 10 en solution dans 250 $cm^3$ de méthanol. On obtient un précipité que l'on dissout par addition de 350 $cm^3$ de méthanol en maintenant la température et l'agitation pendant 1 heure 30.

On neutralise avec une solution d'acide chlorhydrique 0,1N à la même température. On élimine le méthanol, filtre le précipité qui est chromatographié sur silice en éluant avec du cyclohexane et de l'acétate d'éthyle (60-40). On obtient 1,7 g de produit fondant à 88°C.

*Analyse:* $C_{12}H_{15}NOS$ PM: 221,323

| | | | | |
|---|---|---|---|---|
| Calculé: | C% 65,12 | H% 6,83 | S% 14,49 | N% 6,33 |
| Trouvé: | 65,4 | 6,9 | 14,5 | 6,4 |

*Exemple 12*

*3-(acétylthio) N-(2,3-dihydro 1H-inden-1-yl) propanamide.*

On opère comme indiqué à l'exemple 3 à partir de 3,463 g de 1-amino indane dans 150 $cm^3$ de chlorure de méthylène, de 2,166 g de chlorure d'acide 3-(acétylthio) propanoïque en solution dans 150 $cm^3$ de chlorure de méthylène et préparé comme indiqué au stade A de l'exemple 9. On obtient 3,183 g de produit attendu fondant à 109°C.

*Exemple 13*

*N-(2,3-dihydro 1H-inden-1-yl) 3-mercapto propanamide.*

On opère comme indiqué à l'exemple 12, à -6°C, à partir de 119 $cm^3$ de soude 0,1N de 3,128 g de produit obtenu à l'exemple 12 dans 170 $cm^3$ de méthanol. Après chromatographie sur silice en éluant par un mélange chlorure de méthylène-acétate d'éthyle (95-5), on obient 2,022 g de produit attendu fondant à 99°C.

*Analyse:* $C_{12}H_{15}NOS$ PM: 221,324

| | | | | |
|---|---|---|---|---|
| Calculé: | C% 65,12 | H% 6,83 | N% 6,33 | S% 14,49 |
| Trouvé: | 65,3 | 6,39 | 6,2 | 14,8 |

*Exemple 14*

*3-(acétylthio) N-(1,2,3,4-tétrahydro-1-naphthyl) propanamide.*

On ajoute en 30 minutes sous argon et sous agitation, entre -7°C et -10°C, 4,418 g de 1,2,3,4-tétrahydro naphthylamine en solution dans 150 $cm^3$ de chlorure de méthylène à 2,5 g de chlorure d'acide 3-(acétylthio) propanoïque en solution dans 150 $cm^3$ de chlorure de méthylène et préparé comme indiqué au stade A de l'exemple 9. Le précipité obtenu est filtré, mis en suspension dans 1 litre d'éther, agité à 30°C pendant 3 heures. Le précipité est filtré et les deux filtrats ainsi obtenus sont réunis. On élimine les solvants sous pression réduite (20°C) et le résidu obtenu est dissous dans 200 $cm^3$ de chlorure de méthylène, lavé avec de l'acide chlorhydrique 0,1N avec une solution de carbonate acide de sodium saturée puis avec de l'eau. On sèche, élimine les solvants sous pression réduite à 30°C, redissout le résidu dans 1 litre d'éther qui est éliminé jusqu'à observation d'un précipité. On laisse 3 heures à 7°C, filtre et obtient un premier jet. On ajoute au filtrat 100 $cm^3$ de pentane, laisse 3 heures à 7°C, filtre et obtient un deuxième jet. Les deux jets sont séchés 16 heures sous $10^{-2}$ mm de mercure.

On obtient 2,432 g de produit attendu à partir du premier jet et 0,905 g de produit attendu à partir du deuxième jet. Les deux jets présentent chacun un Rf de 0,14 en chromatographie sur silice en éluant avec un mélange cyclohexane-acétate d'éthyle (80-20) F=108-109°C.

*Exemple 15*

*3-mercapto N-(1,2,3,4-tétrahydro 1-naphthyl) propanamide.*

On opère comme indiqué à l'exemple 4 à partir de 3,337 g de produit obtenu à l'exemple 15 dans 250 $cm^3$ de méthanol et de 120 $cm^3$ de soude 0,1N. On obtient 1,850 g de produit attendu fondant à 94°C.

*Analyse:* $C_{13}H_{17}NOS$ PM: 235,350

| | | | | |
|---|---|---|---|---|
| Calculé: | C% 66,34 | H% 7,28 | N% 5,95 | S% 13,62 |
| Trouvé: | 66,4 | 7,4 | 6 | 13,5 |

*Exemple 16*

*3-acétylthio N-(5,6,7,8-tétrahydro 1-naphthyl) propanamide.*

On ajoute en 30 minutes sous argon et sous agitation, entre -7°C et -10°C, 2,5 g de chlorure d'acide 3-(acétylthio) propanoïque en solution dans 150 $cm^3$ de chlorure de méthylène et préparé comme indiqué au stade A de l'exemple 9 à 4,418 g de 1-amino-5,6,7,8-tétrahydro naphthyl en solution dans 150 $cm^3$ de chlorure de méthylène. On filtre le précipité formé, élimine les solvants et obtient un résidu A que l'on dissout dans l'éther, une fraction reste sous forme solide B qui est filtré. On lave le filtrat avec une solution d'acide chlorhydrique 0,1N, une solution de biocarbonate de soude saturée, de l'eau puis avec une solution de chlorure de sodium saturée. On sèche, élimine l'éther et obtient 0,750 g de résidu (fraction C). On dissout la fraction solide obtenue précédemment avec du chlorure de méthylène, lave avec une solution d'acide chlorhydrique 0,1N, une solution de bicarbonate de soude saturée puis de l'eau. On sèche, élimine le solvant sous pression réduite. Le résidu est séché sous $10^{-2}$ mm de Hg pendant 16 heures. On obtient 3,4 g de produit brut (fraction D). Les 2 fractions (C et D) sont réunis et dissoutes dans 1 litre d'éther, la solution obtenue est traitée au charbon actif, filtrée et concentrée. On recristallise dans le pentane. On obtient 3,617 g de produit attendu.

*Exemple 17*

*3-mercapto N-(5,6,7,8-tétrahydro 1-naphthyl) propanamide.*

On ajoute en 30 minutes sous agitation et sous argon, entre -5°C et -10°C, 130 $cm^3$ d'une solution de soude 0,1N dans 3,167 g de produit obtenu à l'exemple 16 en solution dans 600 $cm^3$ de méthanol. On laisse 1 heure sous agitation à cette température, ajoute 130 $cm^3$ d'acide chlorhydrique 0,1N, élimine le méthanol, filtre le précipité obtenu, sèche sous $10^{-2}$ mm de Hg pendant 4 heures. On obtient 2,890 g de produit contenant encore du produit de départ. On refait l'hydrolyse sur le précipité dans les mêmes conditions, avec 250 $cm^3$ de méthanol et 130 $cm^3$ de soude 0,1N. On amène la température à 0°C, filtre le précipité. On purifie le produit par chromatographie sur silice successive en éluant avec du chlorure de méthylène puis avec un mélange chlorure de méthylène-cyclohexane (7-3). On obtient 1,170 g de produit attendu fondant à 106°C.

*Analyse:* $C_{13}H_{17}NOS$ PM: 235,350

| | | | | |
|---|---|---|---|---|
| Calculé: | C% 66,34 | H% 7,28 | S% 13,62 | N% 5,95 |
| Trouvé: | 66,5 | 7,5 | 13,4 | 5,9 |

*Exemple 18*

*α-[(acétylthio)méthyl] N-[1-(phénylméthyl) pipéridin-4-yl] benzène propanamide.*

On dissout 2 g de 1-(phénylméthyl) 4-amino pipéridine dans 100 $cm^3$ de chlorure de méthylène puis ajoute 7,19 g de triéthylamine goutte à goutte. On refroidit la solution à −10°C puis ajoute 1,82 g de chlorure d'α-(acétylthiométhyl) benzène propanoyle dissous dans 100 $cm^3$ de chlorure de méthylène. On maintient à −10°C pendant 3 heures, puis laisse remonter la température, évapore le solvant à 25°C, reprend à l'éther éthylique. On filtre, lave la phase organique à l'eau, sèche et évapore le solvant. On obtient 2,73 g de produit attendu que l'on chromatographie sur silice en éluant au mélange acétate d'éthylène-chlorure de méthylène (1-1) à 2% de méthanol. On obtient 1,7 g de produit. F=78°C.

*Exemple 19*

*α-(mercaptométhyl) N-[1-(phénylméthyl) pipéridin-4-yl] benzène propanamide.*

On opère comme indiqué à l'exemple 4 en utilisant 1,7 g de produit obtenu à l'exemple 19 dissous dans 82 $cm^3$ de méthanol et 41 $cm^3$ de soude 0,1N. On obtient après purification par chromatographie sur silice en éluant au mélange acétate d'éthyle-chlorure de méthylène (1-1) à 5% de méthanol, 1,17 g de produit attendu. F=114°C.

*Analyse:* $C_{22}H_{28}N_2OS$: 368,544

| | | | | |
|---|---|---|---|---|
| Calculé: | C% 71,6 | H% 7,6 | N% 7,6 | S% 8,7 |
| Trouvé: | 71,6 | 7,7 | 7,6 | 8,9 |

*Exemple 20*

*α-[(acétylthio)méthyl] N-[(pyridin-3-yl) méthyl] benzène propanamide.*

On mélange 38 g de 3-picolylamine et 350 $cm^3$ d'éther éthylique, refroidit à 0°C et introduit en 30 minutes, 2,8 g de chlorure d'α-(acétylthiométhyl) benzène propanoyle dissous dans 350 $cm^3$ d'éther éthylique. On laisse remonter la température en 1 heure, ajoute 200 $cm^3$ d'eau puis du bicarbonate de sodium jusqu'à obtention de pH 8-9. On décante, lave la phase organique à l'eau, sèche et évapore le solvant. On obtient 3,38 g de produit attendu. F=80°C.

*Exemple 21*

*Chlorhydrate d'α-(mercaptométhyl) N-[(pyridin-3-yl)méthyl] benzène propanamide.*

On opère comme indiqué à l'exemple 4, en utilisant 3,3 g de produit obtenu à l'exemple 21 dissous dans 200 $cm^3$ de méthanol et 100 $cm^3$ de soude 0,1N. On obtient après purification par chromatographie sur silice en éluant au mélange acétate d'éthyle-chlorure de méthylène (9-1), 2,3 g de produit attendu. F=70°C.

On dissout 2 g de produit dans 3 $cm^3$ de chlorure de méthylène, ajoute 300 $cm^3$ d'éther, refroidit à 0°C, rajoute 200 $cm^3$ d'éther puis ajoute en 30 minutes une solution de 3,3 $cm^3$ d'acide chlorhydrique dans l'éther (2,4N), dans 250 $cm^3$ d'éther. On agite pendant 15 minutes, élimine le solvant, lave les cristaux à l'éther et les sèche. On obtient 2,02 g de produit attendu. F⩽50°C.

*Analyse:* $C_{16}H_{18}N_2OS$, HCl: 322,861

Calculé:
C% 59,52 H% 5,93 N% 8,68 S% 9,93 Cl% 10,98
Trouvé:
C% 59,1 H% 5,9 N% 8,7 S% 9,7 Cl% 11,1

*Exemple 22*

*α-[(acétylthio)méthyl] N-[(pyridin-4-yl) méthyl] benzène propanamide.*

On opère comme indiqué à l'exemple 20, en utilisant 2,8 g de 4-picolylamine et 3,34 g de chlorure d'acide. On obtient 3,75 g de produit attendu. F=96°C.

*Exemple 23*

*Chlorhydrate d'α-(mercaptométhyl) N-[(pyridin-4-yl) méthyl] benzène propanamide.*

On opère comme indiqué à l'exemple 4, en utilisant 3,7 g de produit obtenu à l'exemple 23 dissous dans 225 $cm^3$ de méthanol et 115 $cm^3$ de soude 0,1N. On obtient 3,12 g de produit attendu. On dissout 2,5 g de produit dans le minimum de chlorure de méthylène, puis ajoute 1 litre d'éther éthylique. On refroidit à 0°C puis introduit en 30 minutes une solution de 4 $cm^3$ d'acide chlorhydrique dans l'éther (2,4N), dans 500 $cm^3$ d'éther. On agite pendant 15 minutes, évapore le solvant et recristallise le produit dans l'acétonitrile. On obtient 2 g de produit attendu. F≃145-150°C.

*Analyse:* $C_{16}H_{18}N_2OS$, HCl: 322,861

Calculé:
C% 59,52 H% 5,93 N% 8,68 S% 9,93 Cl% 10,98
Trouvé:
C% 59,5 H% 6,0 N% 8,6 S% 9,7 Cl% 11,1

*Exemple 24*

*α-[(acétylthio)méthyl] N-[(pyridin-2-yl)méthyl] benzène propanamide.*

On opère comme indiqué à l'exemple 20, en utilisant 2,8 g de 2-picolylamine et 3,34 g de chlorure d'acide. On obtient 3,98 g de produit attendu. F=57°C.

*Exemple 25*

*Chlorhydrate d'α-(mercaptométhyl) N-[(pyridin-2-yl) méthyl] benzène propanamide.*

On opère comme indiqué à l'exemple 4, en utilisant 3,92 g de produit obtenu à l'exemple 24, dissous dans 250 $cm^3$ de méthanol et 120 $cm^3$ de soude 0,1N. On obtient 3,44 g de la base du produit attendu. On dissout le produit dans 800 $cm^3$ d'éther, filtre, ajoute à 0°C une solution de 5,5 $cm^3$ d'acide chlorhydrique dans l'éther (2,4N) dans 200 $cm^3$ d'éther. On agite pendant 10 minutes, évapore le solvant et recristallise le produit dans

l'acétonitrile. On obtient 2,83 g de produit attendu. F≃135-140°C.

*Analyse:* $C_{16}H_{18}N_2OS$, HCl: 322,861

Calculé:
C% 59,52 H% 5,93 N% 8,68 S% 9,93 Cl% 10,98
Trouvé:
C% 59,6 H% 5,8 N% 8,5 S% 9,7 Cl% 11,1

*Exemple 26*

*Compositions pharmaceutiques.*

On a préparé des comprimés répondant à la formule:

— composé de l'exemple 2 200 mg
— excipient q.s. pour un comprimé terminé à 350 mg

(Détail de l'ecxcipient: lactose, amidon, stéarate de magnesium, talc).

On a préparé des comprimés répondant à la formule:
— composé de l'exemple 21 200 mg
— excipient q.s. pour un comprimé terminé à 350 mg

(Détail de l'excipient: lactose, amidon, stéarate de magnésium, talc).

ETUDE BIOLOGIQUE

1) *Dosage de l'enképhalinase et détermination de l'effet des inhibiteurs.*

L'activité de l'enképhalinase est déterminée dans une fraction membranaire de striatum de rat.

Les striatum sont prélevés sur glace et homogénéisés en tampon TRIS 0,05M pH 7,4 (20 fois le volume). Après une première centrifugation à 1000 g, la fraction particulaire est obtenue à la suite de deux centrifugations de 10 minutes à 20 000 g. Le culot est ensuite mis en suspension dans un tampon TRIS et conservé à 4°C. Le dosage des protéines est effectué selon la méthode au bleu de Comassie.

Après une préincubation de 15 minutes à 25°C, une aliquote de protéines est incubée pendant 15 minutes à 25°C en présence de 20 nanomoles de leucine-enképaline tritiée (sur le premier acide aminé) préalablement purifiée, d'1 mM de puromycine et du produit à tester en tampon TRIS. La réaction d'hydrolyse est stoppée par addition d'acide chlorhydrique 0,2N et l'incubat subit une déprotéinisation par la chaleur (15 minutes à 95°C). Dans ces conditions, la cinétique de la réaction est linéaire.

Les métabolites tritiés obtenus par hydrolyse sont séparés de l'enképaline par chromatographie sur colonne de porapak Q : ils sont élués avec du tampon TRIS tandis que l'enképhaline est retenue sur la colonne et recueillie ensuite en phase éthanolique.

L'activité des différents produits est exprimée en concentrations inhibitrices 50%.

*Résultats*

| Produit de l'exemple | Inhibition de l'enképhalinase CI 50 en $10^{-6}$ M |
|---|---|
| 2 | 0,1 |
| 8 | 1 |
| 21 | 0,08 |
| 23 | 0,08 |
| 25 | 0,1 |

2) *Test d'analgésie sur tissus inflammés*

[variante de la technique de Randall et Selitto (1)].

La technique consiste à rechercher l'effet analgésique chez le rat dont le seuil de sensibilité à la douleur a été abaissé par une inflammation.

Cette dernière est obtenu par injection de carraghénine (0,25 mg/patte) sous l'aponévrose plantaire d'une patte postérieure. La douleur est provoquée par pression mécanique appliquée sur la face dorsale de la patte et augmentée régulièrement au moyen d'un analgésimètre.

Le seuil douloureux est apprécié par la pression nécessaire pour déclencher une réaction de retrait de la patte ou une réaction de vocalisation de l'animal.

Les produits sont administrés par voie buccale 4 heures après l'injection de l'irritant et les mesures du seuil douloureux sont effectuées immédiatement avant l'injection de l'irritant puis une heure après le traitement.

(1) Randall, L.O. and Selitto, J.J. «a method for measurement of analgesic on inflammed tissue. Arch. Int. Pharmacodyn, 1957, 111, 409.

*Resultats*

| Produit de l'exemple | DA en mg/kg/per os |
|---|---|
| 2 | ⩽ 20 |

**Revendications pour les Etats contractants:**
BE, CH, DE, FR, GB, IT, LI, LU, NL, SE

1. Les composés de formule (I):

$$R_1S\text{-}(CH_2)_n\text{-}\underset{\displaystyle R_2}{\underset{|}{CH}}\text{-}\underset{\displaystyle O}{\underset{\|}{C}}\text{-}NH\text{-}(CH_2)_m\text{-}R_3 \qquad (I)$$

dans laquelle $R_1$ représente un atome d'hydrogène ou un radical $-\underset{\displaystyle O}{\underset{\|}{C}}-R'_1$, $R'_1$ étant un radical alcoyle renfermant de 1 à 5 atomes de carbone ou un radical aryle éventuellement substitué par un radical hydroxy, un radical alcoyle renfermant de 1 à 5 atomes de carbone, un radical alcoxy renfermant de 1 à 5 atomes de carbone, le radical nitro ou un atome d'halogène, n représente un nombre entier pouvant varier de 1 à 5, $R_2$ représente un atome d'hydrogène, un radical alcoyle renfermant de 1 à 5 atomes

de carbone, un radical aryle ou arylalcoyle renfermant de 6 à 15 atomes de carbone, éventuellement substitué par un radical alcoyle renfermant de 1 à 5 atomes de carbone, un radical alcoxy renfermant de 1 à 5 atomes de carbone, un radical hydroxyle, un atome d'halogène ou un radical trifluorométhyle, m représente un nombre entier pouvant varier de 0 à 5, $R_3$ représente:

— soit un radical cyclique saturé renfermant de 3 à 12 atomes de carbone, lequel pouvant être accolé à un radical phényle, $R_3$ ne pouvant toutefois représenter un radical cyclohexyle lorsque $R_2$ et $R_1$ représentent à la fois un atome d'hydrogène et que $n = 1$ et $m = 0$;

— soit un radical phényle éventuellement substitué par un ou plusieurs radicaux choisis parmi les radicaux alcoyles renfermant de 1 à 5 atomes de carbone, alcoxy renfermant de 1 à 5 atomes de carbone, hydroxyle, nitro, halogéno, trifluorméthyle et

$-N{<}^{X}_{X'}$ , X et X' représentant un atome d'hydrogène ou un radical alcoyle renfermant de 1 à 5 atomes de carbone ou un radical phényle auquel est accolé un radical cyclique saturé renfermant de 5 à 9 atomes de carbone;

— soit un radical hétérocyclique choisi parmi les radicaux thiazolyle, 4-5-dihydrothiazolyle, pyridinyle, oxazolyle, isoxazolyle, imidazolyle, pyrimidyle, tétrazolyle, benzimidazolyle, benzothiazolyle, benzoxazolyle, éventuellement substitués par un radical alcoyle renfermant de 1 à 5 atomes de carbone, ou pipéridinyle substitué par un radical aralcoyle renfermant de 7 à 15 atomes de carbone, à la condition que lorsque $R_2$ représente un radical phényle éventuellement substitué par au moins un des radicaux cités précédemment ou lorsque $R_3$ représente un des radicaux hététocycliques mentionnés ci-dessus, éventuellement substitués par un radical alcoyle renfermant de 1 à 5 atomes de carbone, m ne peut représenter la valeur 0 et à la condition que:

a) lorsque $n = 1$ ou 2, $m = 1$, 2 ou 3 et $R_1 = R_2 = H$, $R_3$ ne soit pas un radical phényle éventuellement substitué par un ou deux radicaux alcoyles renfermant de 1 à 5 atomes de carbone,

b) lorsque $m = n = 1$ et $R_1 = CH_3-\underset{\underset{O}{\|}}{C}-$ et $R_2 = CH_3$

$R_3$ ne soit pas un radical phényle, ainsi que leurs sels d'addition avec les acides.

2. Les composés de formule (I), tels que définis à la revendication 1, caractérisés en ce que $R_1$ est un atome d'hydrogène.

3. Les composés de formule (I), tels que définis à la revendication 1, caractérisés en ce que $R_1$ est un radical acétyle.

4. Les composés de formule (I), tels que définis à l'une quelconque des revendications 1 à 3, caractérisés en ce que $n = 1$ et $R_2$ est un radical benzyle ou un atome d'hydrogène.

5. Les composés de formule (I), tels que définis à l'une quelconque des revendications 1 à 4, caractérisés en ce que $m = 0$, 1 ou 2 et $R_3$ représente un radical cyclique saturé de 3 à 8 atomes de carbone éventuellement accolé à un radical phényle ou $R_3$ représente un radical phényle, auquel est accolé un radical cyclique saturé renfermant de 5 à 9 atomes de carbone.

6. Les composés de formule (I) tels que définis à l'une quelconque des revendications 1 à 4, caractérisés en ce que $m = 1$ ou 2 et $R_3$ représente un radical phényle ou pyridinyle.

7. Les composés de formule (I), tels que définis à l'une quelconque des revendications 1 à 6, dont les noms suivent:

— le N-cyclohexyl α-(mercapto méthyl ) benzène propanamide,
— l'α-(mercapto méthyl) N-(2 phényl éthyl) benzène propanamide,
— le N-(2,3-dihydro 1H-inden-2-yl) 3-mercapto propanamide,
— l'α-(mercaptométhyl) N-(2-pyridinylméthyl) benzène propanamide,
— l'α-(mercaptométhyl) N-(3-pyridinylméthyl) benzène propanamide,
— l'α-(mercaptométhyl) N-(4-pyridinylméthyl) benzène propanamide, ainsi que leurs sels d'addition avec les acides.

8. Procédé de préparation des produits de formule (I), dans laquelle $R_1$, $R_2$, $R_3$ et m ont les significations données à la revendication 1 dans laquelle $n = 1$, caractérisé en ce que l'on soumet un acide de formule (II):

$$R'_1-\overset{\overset{O}{\|}}{C}-S-CH_2-\underset{\underset{R_2}{|}}{CH}-COOH \qquad (II)$$

dans laquelle $R'_1$ et $R_2$ ont les significations déjà indiquées, ou un dérivé fonctionnel de cet acide, à l'action d'un produit de formule (III):

$$H_2N-(CH_2)_m-R_3 \qquad (III)$$

dans laquelle $R_3$ et m ont les significations déjà indiquées, pour obtenir un produit de formule (I), dans laquelle $R_1 = R'_1-\underset{\underset{O}{\|}}{C}-$, $R'_1$ ainsi que $R_2$, $R_3$ et m ayant les significations déjà indiquées et dans laquelle $n = 1$, produits de formule (I) que l'on saponifie, le cas échéant, pour obtenir un produit de formule (I) dans laquelle $R_1$ représente un atome d'hydrogène et produits de formule (I) que l'on salifie, le cas échéant, par action d'un acide.

9. Procédé de préparation des produits de formule (I), dans laquelle $R_1$, $R_2$, $R_3$ et m ont les significations données à la revendication 1 et dans laquelle n représente un nombre entier pouvant varier de 1 à 5, caractérisé en ce que l'on soumet un acide de formule (IV):

$$X-(CH_2)_n-\overset{\overset{R_2}{|}}{CH}-COOH \qquad (IV)$$

dans laquelle X est un atome d'halogène, et dans

laquelle n et $R_2$ ont les significations données ci-dessus, ou un dérivé fonctionnel de celui-ci, à l'action dun produit de formule (III) $H_2N(CH_2)_mR_3$ tel que défini à la revendication 8, pour obtenir un produit de formule (V):

$R_2$
|
$X\text{-}(CH_2)_n\text{-}CH\text{-}COHN\text{-}(CH_2)_m\text{-}R_3$ (V)

dans laquelle X, n, m, $R_2$ et $R_3$ ont les significations précédentes, que l'on soumet à l'action de l'anion d'un thioacide de formule (VI):

$R'_1\text{-}C\text{-}SH$ (VI)
||
O

dans laquelle $R'_1$ est défini comme à la revendication 8, pour obtenir un produit de formule (I) dans laquelle $R_1 = R'_1\text{-}C\text{-}$, $R'_1$, $R_2$, $R_3$, n et m ayant les significa-
||
O
tions données ci-dessus, produit de formule (I) que l'on saponifie, le cas échéant, pour obtenir un produit de formule (I) dans laquelle $R_1$ représente un atome d'hydrogène et produits de formule (I) que l'on salifie, le cas échéant, par action d'un acide.

10. A titre de médicaments, les produits de formule (I), tels que définis à l'une quelconque des revendications 1 à 6, ainsi que leurs sels d'addition avec les acides pharmaceutiquement acceptables.

11. A titre de médicaments, les produits tels que définis à la revendication 7, ainsi que leurs sels d'addition avec les acides pharmaceutiquement acceptables.

12. Les compositions pharmaceutiques contenant à titre de principe actif, l'un au moins des médicaments tels que définis aux revendications 10 et 11.

**Revendications pour l'Etat contractant:** AT

1. Procédé pour préparer les composés de formule (I):

$R_1S\text{-}(CH_2)_n\text{-}CH\text{-}C\text{-}NH\text{-}(CH_2)_m\text{-}R_3$ (I)
| ||
$R_2$ O

dans laquelle $R_1$ représente un atome d'hydrogène ou un radical $\text{-}C\text{-}R'_1$, $R'_1$ étant un radical alcoyle ren-
||
O
fermant de 1 à 5 atomes de carbone ou un radical aryle éventuellement substitué par un radical hydroxy, un radical alcoyle renfermant de 1 à 5 atomes de carbone, un radical alcoxy renfermant de 1 à 5 atomes de carbone, le radical nitro ou un atome d'halogène, n représente un nombre entier pouvant varier de 1 à 5, $R_2$ représente un atome d'hydrogène, un radical alcoyle renfermant de 1 à 5 atomes de carbone, un radical aryle ou arylalcoyle renfermant de 6 à 15 atomes de carbone, éventuellement substitué par un radical alcoyle renfermant de 1 à 5 atomes de carbone, un radical alcoxy renfermant de 1 à 5 atomes de carbone, un radical hydroxyle, un atome d'halogène ou un radical trifluorométhyle, m représente un nombre entier pouvant varier de 0 à 5, $R_3$ représente:

— soit un radical cyclique saturé renfermant de 3 à 12 atomes de carbone, lequel pouvant être accolé à un radical phényle, $R_3$ ne pouvant toutefois représenter un radical cyclohexyle lorsque $R_2$ et $R_1$ représentent à la fois un atome d'hydrogène et que $n = 1$ et $m = 0$;

— soit un radical phényle éventuellement substitué par un ou plusieurs radicaux choisis parmi les radicaux alcoyles renfermant de 1 à 5 atomes de carbone, alcoxy renfermant de 1 à 5 atomes de carbone, hydroxyle, nitro, halogéno, trifluorméthyle et
X
-N< , X et X' représentant un atome d'hydrogène
X'
ou un radical alcoyle renfermant de 1 à 5 atomes de carbone ou un radical phényle auquel est accolé un radical cyclique saturé renfermant de 5 à 9 atomes de carbone;

— soit un radical hétérocyclique choisi parmi les radicaux thiazolyle, 4-5-dihydrothiazolyle, pyridinyle, oxazolyle, isoxazolyle, imidazolyle, pyrimidyle, tétrazolyle, benzimidazolyle, benzothiazolyle, benzoxazolyle, éventuellement substitués par un radical alcoyle renfermant de 1 à 5 atomes de carbone, ou pipéridinyle substitué par un radical aralcoyle renfermant de 7 à 15 atomes de carbone, à la condition que lorsque $R_3$ représente un radical phényle éventuellement substitué par au moins un des radicaux cités précédemment ou lorsque $R_3$ représente un des radicaux hétérocycliques mentionnés ci-dessus, éventuellement substitués par un radical alcoyle renfermant de 1 à 5 atomes de carbone, m ne peut représenter la valeur 0 et à la condition que:

a) lorsque $n = 1$ ou 2, $m = 1$, 2 ou 3 et $R_1 = R_2 = H$, $R_3$ ne soit pas un radical phényle éventuellement substitué par un ou deux radicaux alcoyles renfermant de 1 à 5 atomes de carbone.

b) lorsque $m = n = 1$ et $R_1 = CH_3\text{-}C\text{-}$ et $R_2 = CH_3$
||
O
$R_3$ ne soit pas un radical phényle ainsi que leurs sels d'addition avec les acides, caractérisé en ce que

— pour préparer les composés de formule (I) dans laquelle $R_1$, $R_2$ et $R_3$ et m ont la signification précitée et dans laquelle $n = 1$ ainsi que leurs sels d'addition avec les acides, l'on soumet un acide de formule (II):

O
||
$R'_1\text{-}C\text{-}S\text{-}CH_2\text{-}CH\text{-}COOH$ (II)
|
$R_2$

dans laquelle $R'_1$ et $R_2$ ont les significations déjà indiquées, ou un dérivé fonctionnel de cet acide, à l'action d'un produit de formule (III):

$H_2N\text{-}(CH_2)_m\text{-}R_3$ (III)

dans laquelle $R_3$ et m ont les significations déjà indiquées, pour obtenir un produit de formule (I), dans laquelle $R_1 = R'_1\text{-C(=O)-}$, $R'_1$ ainsi que $R_2$, $R_3$ et m ayant les significations déjà indiquées et dans laquelle n = 1, produits de formule (I) que l'on saponifie, le cas échéant, pour obtenir un produit de formule (I) dans laquelle $R_1$ représente un atome d'hydrogène et produits de formule (I) que l'on salifie, le cas échéant, par action d'un acide pour en former le sel;

— pour préparer les composés de formule (I) dans laquelle $R_1$, $R_2$ et $R_3$ et m ont la signification précitée et dans laquelle n représente un nombre entier pouvant varier de 1 à 3, l'on soumet un acide de formule (IV):

$$X\text{-}(CH_2)_n\text{-}CH(R_2)\text{-}COOH \qquad (IV)$$

dans laquelle X est un atome d'halogène, et dans laquelle n et $R_2$ ont les significations données ci-dessus, ou un dérivé fonctionnel de celui-ci, à l'action d'un produit de formule (III) $H_2N(CH_2)_mR_3$ tel que défini précédemment pour obtenir un produit de formule (V):

$$X\text{-}(CH_2)_n\text{-}CH(R_2)\text{-}COHN\text{-}(CH_2)_m\text{-}R_3 \qquad (V)$$

dans laquelle X, n, m, $R_2$ et $R_3$ ont les significations précédentes, que l'on soumet à l'action de l'anion d'un thioacide de formule (VI):

$$R'_1\text{-}C(=O)\text{-}SH \qquad (VI)$$

dans laquelle $R'_1$ est défini comme précédemment, pour obtenir un produit de formule (I) dans laquelle $R_1 = R'_1\text{-C(=O)-}$, $R'_1$, $R_2$, $R_3$, n et m ayant les significations données ci-dessus, produit de formule (I) que l'on saponifie, le cas échéant, pour obtenir un produit de formule (I) dans laquelle $R_1$ représente un atome d'hydrogène et produits de formule (I) que l'on salifie, le cas échéant, par action d'un acide pour en former le sel.

2. Procédé selon la revendication 1, pour préparer les composés de formule (I) dans laquelle $R_1$, $R_2$ et $R_3$ et n ont la signification précitée et dans laquelle n = 1 ainsi que leurs sels d'addition avec les acides, caractérisé en ce que l'on soumet un acide de formule (II):

$$R'_1\text{-}C(=O)\text{-}S\text{-}CH_2\text{-}CH(R_2)\text{-}COOH \qquad (II)$$

dans laquelle $R'_1$ et $R_2$ ont les significations déjà indiquées, ou un dérivé fonctionnel de cet acide, à l'action d'un produit de formule (III):

$$H_2N\text{-}(CH_2)_m\text{-}R_3 \qquad (III)$$

dans laquelle $R_3$ et m ont les significations déjà indiquées, pour obtenir un produit de formule (I), dans laquelle $R_1 = R'_1\text{-C(=O)-}$, $R'_1$ ainsi que $R_2$, $R_3$ et m ayant les significations déjà indiquées et dans laquelle n = 1, produits de formule (I) que l'on saponifie, le cas échéant, pour obtenir un produit de formule (I) dans laquelle $R_1$ représente un atome d'hydrogène et produits de formule (I) que l'on salifie, le cas échéant, par action d'un acide pour en former le sel.

3. Procédé selon la revendication 1, pour préparer les composés de formule (1), dans laquelle $R_1$, $R_2$ et $R_3$ et m ont la signification précitée et dans laquelle n représente un nombre entier pouvant varier de 1 à 3, caractérisé en ce que l'on soumet un acide de formule (IV):

$$X\text{-}(CH_2)_n\text{-}CH(R_2)\text{-}COOH \qquad (IV)$$

dans laquelle X est un atome d'halogène, et dans laquelle n et $R_2$ ont les significations données ci-dessus, ou un dérivé fonctionnel de celui-ci, à l'action d'un produit de formule (III) $H_2N(CH_2)_mR_3$ tel que défini précédemment, pour obtenir un produit de formule (V):

$$X\text{-}(CH_2)_n\text{-}CH(R_2)\text{-}COHN\text{-}(CH_2)_m\text{-}R_3 \qquad (V)$$

dans laquelle X, n, m, $R_2$ et $R_3$ ont les significations précédentes, que l'on soumet à l'action de l'anion d'un thioacide de formule (VI):

$$R'_1\text{-}C(=O)\text{-}SH \qquad (VI)$$

dans laquelle $R'_1$ est défini comme précédemment pour obtenir un produit de formule (I) dans laquelle $R_1 = R'_1\text{-C(=O)-}$, $R'_1$, $R_2$, $R_3$, n et m ayant les significations données ci-dessus, produit de formule (I) que l'on saponifie, le cas échéant, pour obtenir un produit de formule (I) dans laquelle $R_1$ représente un atome d'hydrogène et produits de formule (I) que l'on salifie, le cas échéant, par action d'un acide pour en former le sel.

4. Procédé selon la revendication 1, pour la préparation des composés de formule (I) telle que définie à la revendication 1, répondant à la formule (I')

$$R_1\text{-}S\text{-}(CH_2)_n\text{-}CH(R_2)\text{-}C(=O)\text{-}NH\text{-}(CH_2)_m\text{-}R'_3 \qquad (I')$$

dans laquelle $R_1$, $R_2$, n et m conservent leurs significations précitées et $R'_3$ représente

— soit un radical cyclique saturé renfermant de 3 à 12 atomes de carbone, lequel pouvant être accolé à un radical phényle, $R_3$ ne pouvant toutefois représenter un radical cyclohexyle lorsque $R_2$ et $R_1$ représentent à la fois un atome d'hydrogène et que $n=1$ et $m=0$;

— soit un radical phényle éventuellement substitué par un ou plusieurs radicaux choisis parmi les radicaux alcoyles renfermant de 1 à 5 atomes de carbone, alcoxy renfermant de 1 à 5 atomes de carbone, hydroxyle, nitro, halogéno, trifluorméthyle et $-N\begin{matrix} X \\ X' \end{matrix}$, X et X' représentant un atome d'hydrogène ou un radical alcoyle renfermant de 1 à 5 atomes de carbone ou un radical phényle auquel est accolé un radical cyclique saturé renfermant de 5 à 9 atomes de carbone;

— soit un radical hétérocyclique choisi parmi les radicaux thiazolyle, 4-5-dihydrothiazole, pyridinyle, oxazolyle, isoxazolyle, imidazolyle, pyrimidyle, tétrazolyle, benzimidazolyle, benzothiazolyle, benzoxazolyle, éventuellement substitués par un radical alcoyle renfermant de 1 à 5 atomes de carbone, ou pipéridinyle substitué par un radical aralcoyle renfermant de 7 à 15 atomes de carbone, à la condition que lorsque $R'_3$ représente un radical phényle éventuellement substitué par au moins un des radicaux cités précédemment ou lorsque $R'_3$ représente un des radicaux hétérocycliques mentionnés ci-dessus, éventuellement substitués par un radical alcoyle renfermant de 1 à 5 atomes de carbone, m ne peut représenter la valeur 0 et à la condition que:

a) lorsque $n=1$ ou 2, $m=1$, 2 ou 3 et $R_1=R_2=H$, $R'_3$ ne soit un radical phényle éventuellement substitué par un ou deux radicaux alcoyles renfermant de 1 à 5 atomes de carbone,

b) lorsque $m=n=1$ et $R_1=CH_3\text{-}\underset{\underset{O}{\|}}{C}\text{-}$ et $R_2=CH_3$, $R'_3$ ne soit pas un radical phényle ainsi que leurs sels d'addition avec les acides, caractérisé en ce que l'on utilise au départ un produit de formule (III) dans laquelle $R_3$ a les valeurs de $R'_3$ indiquées ci-dessus.

5. Procédé selon la revendication 4, caractérisé en ce que l'on utilise au départ un produit de formule (II) ou (VI) dans laquelle $R'_1\text{-}\underset{\underset{O}{\|}}{C}\text{-}$ représente un radical acétyle.

6. Procédé selon la revendication 5, caractérisé en ce que l'on utilise au départ un produit de formule (V) ou (IV) dans laquelle $R_2$ représente un radical benzyle ou un atome d'hydrogène et n est égal à 1.

7. Procédé selon l'une quelconque des revendications 4 à 6, caractérisé en ce que l'on utilise au départ un produit de formule (III) dans laquelle $m=0$, 1 ou 2, $R_3$ représente un radical cyclique saturé de 3 à 8 atomes de carbone éventuellement accolé à un radical phényle ou $R_3$ représente un radical phényle auquel est accolé un radical cyclique saturé renfermant de 5 à 9 atomes de carbone.

8. Procédé selon l'une quelconque des revendications 4 à 6, caractérisé en ce que l'on utilise au départ un produit de formule (III) dans laquelle $m=1$ ou 2 et $R_3$ représente un radical phényle ou pyridinyle.

**Patentansprüche für die Vertragsstaaten:**
BE, CH, DE, FR, GB, IT, LI, LU, NL, SE

1. Verbindungen der Formel (I)

$$R_1S\text{-}(CH_2)_n\text{-}\underset{\underset{R_2}{|}}{CH}\text{-}\underset{\underset{O}{\|}}{C}\text{-}NH\text{-}(CH_2)_m\text{-}R_3 \qquad (I)$$

worin $R_1$ ein Wasserstoffatom oder einen Rest $\text{-}\underset{\underset{O}{\|}}{C}\text{-}R'_1$ darstellt, worin $R'_1$ einen Alkylrest mit 1 bis 5 Kohlenstoffatomen oder einen Arylrest, der gegebenenfalls substituiert ist durch eine Hydroxygruppe, einen Alkylrest mit 1 bis 5 Kohlenstoffatomen, eine Alkoxygruppe mit 1 bis 5 Kohlenstoffatomen, die Nitrogruppe oder ein Halogenatom, bedeutet, n eine ganze Zahl von 1 bis 5 dargestellt, $R_2$ für ein Wasserstoffatom, einen Alkylrest mit 1 bis 5 Kohlenstoffatomen, einen Aryl- oder Aralkylrest mit 6 bis 15 Kohlenstoffatomen, der gegebenenfalls durch einen Alkylrest mit 1 bis 5 Kohlenstoffatomen, eine Hydroxylgruppe, ein Halogenatom oder eine Trifluormethylgruppe substituiert ist, steht, m eine ganze Zahl von 0 bis 5 bedeutet, $R_3$ bedeutet:

— entweder einen gesättigten, cyclischen Rest mit 3 bis 12 Kohlenstoffatomen, der mit einem Phenylrest verknüpft sein kann, wobei $R_3$ jedoch keinen Cyclohexylrest darstellen kann, wenn $R_2$ und $R_1$ gleichzeitig ein Wasserstoffatom bedeuten und $n=1$ und $m=0$;

— oder einen Phenylrest, der gegebenenfalls durch einen oder mehrere Reste, ausgewählt unter den Alkylresten mit 1 bis 5 Kohlenstoffatomen, den Alkoxyresten mit 1 bis 5 Kohlenstoffatomen, der Hydroxyl-, Nitro-Gruppe, Halogen, Trifluormethyl und $-N\begin{matrix} X \\ X' \end{matrix}$, substituiert sein kann, wobei X und X' ein Wasserstoffatom oder einen Alkylrest mit 1 bis 5 Kohlenstoffatomen darstellen oder einen Phenylrest, an den ein gesättigter, cyclischer Rest mit 5 bis 9 Kohlenstoffatomen geknüpft ist;

— oder einen heterocyclischen Rest, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Benzimidazolyl, Benzothiazolyl-, Benzoxazolylresten, die gegebenenfalls durch einen Alkylrest mit 1 bis 5 Kohlenstoffatomen substituiert sind, oder dem Piperidinylrest, der durch einen Aralkylrest mit 7 bis 15 Kohlenstoffatomen substituiert ist, unter der Voraussetzung, dass, wenn $R_3$ einen gegebenenfalls durch zumindest einen der vorstehenden Reste substituierten Phenylrest darstellt oder wenn $R_3$ einen der vorstehend erwähnten heterocyclischen Reste, die gegebenenfalls durch einen Alkylrest mit 1 bis 5 Kohlenstoffatomen substituiert sind, bedeutet, m nicht den Wert 0 annehmen kann, und unter der Voraussetzung, dass,

a) wenn $n = 1$ oder 2, $m = 1$, 2 oder 3 und $R_1 = R_2 = H$, $R_3$ nicht einen gegebenenfalls durch einen oder zwei Alkylreste mit 1 bis 5 Kohlenstoffatomen substituierten Phenylrest darstellen kann,

b) wenn $m = n = 1$ und $R_1 = CH_3\text{-}\overset{\displaystyle O}{\overset{\|}{C}}$ und $R_2 = CH_3$, $R_3$ keinen Phenylrest darstellen kann, sowie deren Additionssalze mit Säuren.

2. Verbindungen der Formel (I) gemäss Anspruch 1, dadurch gekennzeichnet, dass $R_1$ ein Wasserstoffatom ist.

3. Verbindungen der Formel (I) gemäss Anspruch 1, dadurch gekennzeichnet, dass $R_1$ ein Acetylrest ist.

4. Verbindungen der Formel (I) gemäss einem der Ansprüche 1 bis 3, dadurch gekennzeichnet, dass $n = 1$ und $R_2$ ein Benzylrest oder ein Wasserstoffatom ist.

5. Verbindungen der Formel (I) gemäss einem der Ansprüche 1 bis 4, dadurch gekennzeichnet, dass $m = 0$, 1 oder 2 und $R_3$ einen gesättigten, cyclischen Rest mit 3 bis 8 Kohlenstoffatomen, der gegebenenfalls an einen Phenylrest geknüpft ist, bedeutet oder $R_3$ einen Phenylrest bedeutet, an den ein gesättigter, cyclischer Rest mit 5 bis 9 Kohlenstoffatomen geknüpft ist.

6. Verbindungen der Formel (I) gemäss einem der Ansprüche 1 bis 4, dadurch gekennzeichnet, dass $m = 1$ oder 2 und $R_3$ einen Phenyl- oder Pyridinylrest bedeutet.

7. Verbindungen der Formel (I) gemäss einem der Ansprüche 1 bis 6 mit den folgenden Bezeichnungen:

N-Cyclohexyl-α-(mercaptomethyl)-benzolpropanamid,

α-(mercaptomethyl)-N-(2-phenylethyl)-benzolpropanamid,

N-(2,3-Dihydro-1H-inden-2-yl)-3-mercaptopropanamid,

α-(Mercaptomethyl)-N-(2-pyridinylmethyl)-benzolpropanamid,

α-(Mercaptomethyl)-N-(3-pyridinylmethyl)-benzolpropanamid,

α-(Mercaptomethyl)-N-(4-pyridinylmethyl)-benzolpropanamid

sowie deren Additionssalze mit Säuren.

8. Verfahren zur Herstellung der Produkte der Formel (I), worin $R_1$, $R_2$, $R_3$ und m die in Anspruch 1 angegebenen Bedeutungen besitzen und $n = 1$, dadurch gekennzeichnet, dass man eine Säure der Formel (II)

$$R'_1\text{-}\overset{\displaystyle O}{\overset{\|}{C}}\text{-}S\text{-}CH_2\text{-}\underset{\displaystyle R_2}{\underset{|}{CH}}\text{-}COOH \qquad (II)$$

worin $R'_1$ und $R_2$ die angegebenen Bedeutungen besitzen, oder ein funktionelles Derivat dieser Säure der Einwirkung eines Produkts der Formel (III)

$$H_2N\text{-}(CH_2)_m\text{-}R_3 \qquad (III)$$

worin $R_3$ und m die angegebenen Bedeutungen besitzen, unterzieht, um ein Produkt der Formel (I) zu erhalten, worin $R_1 = R'_1\text{-}\overset{\displaystyle O}{\overset{\|}{C}}\text{-}$, wobei $R'_1$ sowie $R_2$, $R_3$ und m die angegebenen Bedeutungen besitzen und $n = 1$, die Produkte der Formel (I) gegebenenfalls verseift, um ein Produkt der Formel (I) zu erhalten, worin $R_1$ ein Wasserstoffatom bedeutet, und die Produkte der Formel (I) gegebenenfalls durch ein Einwirken einer Säure in ein Salz überführt.

9. Verfahren zur Herstellung der Produkte der Formel (I), worin $R_1$, $R_2$, $R_3$ und m die in Anspruch 1 angegebenen Bedeutungen besitzen und worin n eine ganze Zahl von 1 bis 5 bedeutet, dadurch gekennzeichnet, dass man eine Säure der Formel (IV)

$$X\text{-}(CH_2)_n\text{-}\overset{\displaystyle R_2}{\overset{|}{CH}}\text{-}COOH \qquad (IV)$$

worin X ein Halogenatom darstellt und worin n und $R_2$ die vorstehend angegebenen Bedeutungen besitzen, oder ein funktionelles Derivat dieser Säure der Einwirkung eines Produkts der Formel (III) $H_2N(CH_2)_mR_3$, wie in Anspruch 8 definiert, unterzieht, um ein Produkt der Formel (V)

$$X\text{-}(CH_2)_n\text{-}\overset{\displaystyle R_2}{\overset{|}{CH}}\text{-}COHN\text{-}(CH_2)_m\text{-}R_3 \qquad (V)$$

zu erhalten, worin X, n, m, $R_2$ und $R_3$ die vorstehenden Bedeutungen besitzen, das man der Einwirkung eines Anions einer Thiosäure der Formel (VI)

$$R_1'\text{-}\underset{\displaystyle O}{\underset{\|}{C}}\text{-}SH \qquad (VI)$$

unterzieht, worin $R'_1$ wie in Anspruch 8 definiert ist, um ein Produkt der Formel (I) zu erhalten, worin $R_1 = R'_1\text{-}\underset{\displaystyle O}{\underset{\|}{C}}\text{-}$, $R'_1$, $R_2$, $R_3$, n und m die vorstehenden Bedeutungen besitzen, das Produkt der Formel (I) gegebenenfalls verseift, um ein Produkt der Formel (I) zu erhalten, worin $R_1$ ein Wasserstoffatom bedeutet, und die Produkte der Formel (I) gegebenenfalls durch Einwirken einer Säure in ein Salz überführt.

10. Als Arzneimittel die Produkte der Formel (I) gemäss einem der Ansprüche 1 bis 6 sowie deren Additionssalze mit pharmazeutisch verträglichen Säuren.

11. Als Arzneimittel die Produkte, wie in Anspruch 7 definiert, sowie deren Additionssalze mit pharmazeutisch verträglichen Säuren.

12. Pharmazeutische Zusammensetzungen, enthaltend als Wirkstoff zumindest eines der Arzneimittel gemäss einem der Ansprüche 10 und 11.

**Patentansprüche für den Vertragsstaat:** AT

1. Verfahren zur Herstellung der Verbindungen der Formel (I)

$$R_1S\text{-}(CH_2)_n\text{-}\underset{\displaystyle R_2}{\underset{|}{CH}}\text{-}\underset{\displaystyle O}{\underset{\|}{C}}\text{-}NH\text{-}(CH_2)_m\text{-}R_3 \qquad (I)$$

worin $R_1$ ein Wasserstoffatom oder einen Rest $-\underset{\underset{O}{\|}}{C}-R'_1$ darstellt, worin $R'_1$ einen Alkylrest mit 1 bis 5 Kohlenstoffatomen oder einen Arylrest, der gegebenenfalls substituiert ist durch eine Hydroxygruppe, einen Alkylrest mit 1 bis 5 Kohlenstoffatomen, eine Alkoxygruppe mit 1 bis 5 Kohlenstoffatomen, die Nitrogruppe oder ein Halogenatom, bedeutet, n eine ganze Zahl von 1 bis 5 darstellt, $R_2$ für ein Wasserstoffatom, einen Alkylrest mit 1 bis 5 Kohlenstoffatomen, einen Aryl- oder Aralkylrest mit 6 bis 15 Kohlenstoffatomen, der gegebenenfalls durch einen Alkylrest mit 1 bis 5 Kohlernstoffatomen, eine Alkoxygruppe mit 1 bis 5 Kohlenstoffatomen, eine Hydroxylgruppe, ein Halogenatom oder eine Trifluormethylgruppe substituiert ist, steht, m eine ganze Zahl von 0 bis 5 bedeutet, $R_3$ bedeutet:

— entweder einen gesättigten, cyclischen Rest mit 3 bis 12 Kohlenstoffatomen, der mit einem Phenylrest verknüpft sein kann, wobei $R_3$ jedoch keinen Cyclohexylrest darstellen kann, wenn $R_2$ und $R_1$ gleichzeitig ein Wasserstoffatom bedeuten und $n = 1$ und $m = 0$;

— oder einen Phenylrest, der gegebenenfalls durch einen oder mehrere Reste, ausgewählt unter den Alkylresten mit 1 bis 5 Kohlenstoffatomen, den Alkoxyresten mit 1 bis 5 Kohlenstoffatomen, der Hydroxyl-, Nitro-Gruppe, Halogen, Trifluomethyl und $-N\langle^{X}_{X'}$, substituiert sein kann, wobei X und X' ein Wasserstoffatom oder einen Alkylrest mit 1 bis 5 Kohlenstoffatomen darstellen oder einen Phenylrest, an den ein gesättigter, cyclischer Rest mit 5 bis 9 Kohlenstoffatomen geknüpft ist;

— oder einen heterocyclischen Rest, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Benzimidazolyl-, Benzothiazolyl-, Benzoxazolylresten, die gegebenenfalls durch einen Alkylrest mit 1 bis 5 Kohlenstoffatomen substituiert sind, oder dem Piperidinylrest, der durch einen Aralkylrest mit 7 bis 15 Kohlenstoffatomen substituiert ist, unter der Voraussetzung, dass, wenn $R_3$ einen gegebenenfalls durch zumindest einen der vorstehenden Reste substituierten Phenylrest darstellt oder wenn $R_3$ einen der vorstehend erwähnten heterocyclischen Reste, die gegebenenfalls durch einen Alkylrest mit 1 bis 5 Kohlenstoffatomen substituiert sind, bedeutet, m nicht den Wert 0 annehmen kann, und unter der Voraussetzung, dass,

a) wenn $n = 1$ oder 2, $m = 1$, 2 oder 3 und $R_1 = R_2 = H$, $R_3$ nicht einen gegebenenfalls durch einen oder zwei Alkylreste mit 1 bis 5 Kohlenstoffatomen substituierten Phenylrest darstellen kann,

b) wenn $m = n = 1$ und $R_1 = CH_3-\underset{\underset{O}{\|}}{C}-$ und $R_2 = CH_3$, $R_3$ keinen Phenylrest darstellen kann,

sowie von deren Additionssalzen mit Säuren, dadurch gekennzeichnet, dass man

— zur Herstellung der Verbindungen der Formel (I), worin $R_1$, $R_2$, $R_3$ und m die vorstehende Bedeutung besitzen und worin $n = 1$, sowie von deren Additionssalzen mit Säuren eine Säure der Formel (II)

$$R'_1-\overset{\overset{O}{\|}}{C}-S-CH_2-\underset{\underset{R_2}{|}}{CH}-COOH \qquad (II)$$

worin $R'_1$ und $R_2$ die angegebenen Bedeutungen besitzen, oder ein funktionelles Derivat dieser Säure der Einwirkung eines Produkts der Formel (III)

$$H_2N-(CH_2)_m-R_3 \qquad (III)$$

unterzieht, worin $R_3$ und m die angegebenen Bedeutungen besitzen, um ein Produkt der Formel (I) zu erhalten, worin $R_1 = R'_1-\underset{\underset{O}{\|}}{C}-$, $R'_1$ sowie $R_2$, $R_3$ und m die angegebenen Bedeutungen besitzen und worin $n = 1$, die Produkte der Formel (I) gegebenenfalls verseift, um ein Produkt der Formel (I) zu erhalten, worin $R_1$ ein Wasserstoffatom bedeutet, und die Produkte der Formel (I) gegebenenfalls durch Einwirken einer Säure, um hieraus das Salz zu bilden, in ein Salz überführt;

— zur Herstellung der Verbindungen der Formel (I), worin $R_1$, $R_2$, $R_3$ und m die vorstehende Bedeutung haben und worin n eine ganze Zahl von 1 bis 3 bedeutet eine Säure der Formel (IV)

$$X-(CH_2)_n-\overset{\overset{R_2}{|}}{CH}-COOH \qquad (IV)$$

worin X ein Halgenatom darstellt und worin n und $R_2$ die vorstehenden Bedeutungen besitzen, oder ein funktionelles Derivat dieser Säure der Einwirkung eines Produkts der Formel (III) $H_2N(CH_2)_mR_3$, wie vorstehend definiert, unterzieht, um ein Produkt der Formel (V)

$$X-(CH_2)_n-\overset{\overset{R_2}{|}}{CH}-COHN-(CH_2)_m-R_3 \qquad (V)$$

zu erhalten, worin X, n, m, $R_2$ und $R_3$ die vorstehenden Bedeutungen besitzen, das man der Einwirkung eines Anions einer Thiosäure der Formel (VI)

$$R'_1-\underset{\underset{O}{\|}}{C}-SH \qquad (VI)$$

unterzieht, worin $R'_1$ wie vorstehend definiert ist, um ein Produkt der Formel (I) zu erhalten, worin $R_1 = R'_1-\underset{\underset{O}{\|}}{C}-$, $R'_1$, $R_2$, $R_3$, n und m die vorstehenden Bedeutungen besitzen, das Produkt der Formel (I) gegebenenfalls verseift, um ein Produkt der Formel (I) zu erhalten, worin $R_1$ ein Wasserstoffatom bedeutet, und die Produkte der Formel (I) gegebenenfalls durch Einwirken einer Säure, um hieraus das Salz zu bilden, in ein Salz überführt.

2. Verfahren gemäss Anspruch 1, zur Herstellung von Verbindungen der Formel (I), worin $R_1$, $R_2$, $R_3$ und m die vorstehende Bedeutung besitzen und worin n = 1, sowie von deren Additionssalzen mit Säuren, dadurch gekennzeichnet, dass man eine Säure der Formel (II)

$$R'_1-\underset{\displaystyle \|}{\overset{O}{}}\!\!\overset{\displaystyle O}{\overset{\|}{C}}-S-CH_2-\underset{\displaystyle R_2}{\underset{|}{CH}}-COOH \qquad (II)$$

worin $R'_1$ und $R_2$ die vorstehenden Bedeutungen besitzen, oder ein funktionelles Derivat dieser Säure der Einwirkung eines Produkts der Formel (III)

$$H_2N-(CH_2)_m-R_3 \qquad (III)$$

unterzieht, worin $R_3$ und m die angegebenen Bedeutungen besitzen, um ein Produkt der Formel (I) zu erhalten, worin $R_1 = R'_1-\underset{\displaystyle O}{\underset{\|}{C}}-$, $R'_1$ sowie $R_2$, $R_3$ und m die angegebenen Bedeutungen besitzen und worin n = 1, die Produkte der Formel (I) gegebenenfalls verseift, um ein Produkt der Formel (I) zu erhalten, worin $R_1$ ein Wasserstoffatom bedeutet, und die Produkte der Formel (I) gegebenenfalls durch Einwirken einer Säure, um hieraus das Salz zu bilden, in ein Salz überführt.

3. Verfahren gemäss Anspruch 1 zur Herstellung von Verbindungen der Formel (I), worin $R_1$, $R_2$, $R_3$ und m die vorstehende Bedeutung besitzen und worin n eine ganze Zahl von 1 bis 3 darstellt, dadurch gekennzeichnet, dass man eine Säure der Formel (IV)

$$X-(CH_2)_n-\overset{\displaystyle R_2}{\overset{|}{CH}}-COOH \qquad (IV)$$

worin X ein Halogenatom bedeutet und worin $R_2$ und n die vorstehenden Bedeutungen besitzen, oder ein funktionelles Derivat dieser Säure der Einwirkung eines Produkts der Formel (III) $H_2N(CH_2)_mR_3$, wie vorstehend definiert, unterzieht, um ein Produkt der Formel (V)

$$X-(CH_2)_n-\overset{\displaystyle R_2}{\overset{|}{CH}}-COHN-(CH_2)_m-R_3 \qquad (V)$$

zu erhalten, worin X, n, m, $R_2$ und $R_3$ die angegebenen Bedeutungen besitzen, das man der Einwirkung eines Anions einer Thiosäure der Formel (VI)

$$R'_1-\underset{\displaystyle O}{\underset{\|}{C}}-SH \qquad (VI)$$

unterzieht, worin $R'_1$ wie vorstehend definiert ist, um ein Produkt der Formel (I) zu erhalten, worin $R_1 = R'_1-\underset{\displaystyle O}{\underset{\|}{C}}-$, $R'_1$, $R_2$, $R_3$, n und m die vorstehenden Bedeutungen besitzen, das Produkt der Formel (I) gegebenenfalls verseift, um ein Produkt der Formel (I) zu erhalten, worin $R_1$ ein Wasserstoffatom bedeutet, und die Produkte der Formel (I) gegebenenfalls durch Einwirken einer Säure, um hieraus das Salz zu bilden, in ein Salz überführt.

4. Verfahren gemäss Anspruch 1 zur Herstellung von Verbindungen der Formel (I) gemäss Anspruch 1 der Formel (I')

$$R_1-S-(CH_2)_n-\underset{\displaystyle R_2}{\underset{|}{CH}}-\underset{\displaystyle O}{\underset{\|}{C}}-NH-(CH_2)_m-R'_3 \qquad (I')$$

worin $R_1$, $R_2$, n und m die vorstehenden Bedeutungen besitzen und $R'_3$ bedeutet:

— entweder einen gesättigten, cyclischen Rest mit 3 bis 12 Kohlenstoffatomen, der an einen Phenylrest geknüpft sein kann, wobei $R_3$ jedoch nicht einen Cyclohexylrest bedeuten kann, wenn $R_2$ und $R_1$ gleichzeitig ein Wasserstoffatom bedeuten, und n = 1 und m = 2;

— oder einen Phenylrest, der gegebenenfalls durch einen oder mehrere Reste, ausgewählt unter den Alkylresten mit 1 bis 5 Kohlenstoffatomen, den Alkoxyresten mit 1 bis 5 Kohlenstoffatomen, der Hydroxylgruppe, der Nitrogruppe, Halogen, Trifluormethyl und $-N\langle{}^{X}_{X'}$, substituiert sein kann, wobei X und X' ein Wasserstoffatom oder einen Alkylrest mit 1 bis 5 Kohlenstoffatomen oder einen Phenylrest bedeuten, an den ein gesättigter, cyclischer Rest mit 5 bis 9 Kohlenstoffatomen geknüpft ist;

— oder einen heterocyclischen Rest, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Benzimidazolyl-, Benzothiazolyl-, Benzoxazolyl-Resten, die gegebenenfalls durch einen Alkylrest mit 1 bis 5 Kohlenstoffatomen substituiert sind, oder dem durch einen Aralkylrest mit 7 bis 15 Kohlenstoffatomen substituierten Piperidinylrest, unter der Bedingung, dass, wenn $R'_3$ einen gegebenenfalls durch zumindest einen der vorstehenden Reste substituierten Phenylrest bedeutet oder $R'_3$ einen der vorstehend erwähnten heterocyclischen Reste, die gegebenenfalls durch einen Alkylrest mit 1 bis 5 Kohlenstoffatomen substituiert sind, bedeutet, m nicht den Wert 0 annehmen kann, und unter der Bedingung, dass

a) wenn n = 1 oder 2, m = 1, 2 oder 3 und $R_1 = R_2 = H$, $R'_3$ keinen gegebenenfalls durch einen oder zwei Alkylreste mit 1 bis 5 Kohlenstoffatomen substituierten Phenylrest bedeutet,

b) wenn m = n = 1 und $R_1 = CH_3-\underset{\displaystyle O}{\underset{\|}{C}}-$ und $R_2 = CH_3$, $R'_3$ keinen Phenylrest bedeutet, sowie von deren Additionssalzen mit Säuren, dadurch gekennzeichnet, dass man von einem Produkt der Formel (III) ausgeht, worin $R_3$ die vorstehend für $R'_3$ angegebenen Bedeutungen besitzt.

5. Verfahren gemäss Anspruch 4, dadurch gekennzeichnet, dass man von einem Produkt der For-

mel (II) oder (VI) ausgeht, worin $R'_1$-C- einen Acetylrest bedeutet.

$$R'_1\text{-}\underset{\displaystyle O}{\overset{}{\underset{\|}{C}}}\text{-}$$

6. Verfahren gemäss Anspruch 5, dadurch gekennzeichnet, dass man von einem Produkt der Formel (V) oder (IV) ausgeht, worin $R_2$ einen Benzylrest oder ein Wasserstoffatom bedeutet und n für 1 steht.

7. Verfahren gemäss einem der Ansprüche 4 bis 6, dadurch gekennzeichnet, dass man von einem Produkt der Formel (III) ausgeht, worin m = 0, 1 oder 2, $R_3$ einen gesättigten, cyclischen Rest mit 3 bis 8 Kohlenstoffatomen, der gegebenenfalls an einen Phenylrest geknüpft ist, bedeutet oder $R_3$ einen Phenylrest bedeutet, an den ein gesättigter, cyclischer Rest mit 5 bis 9 Kohlenstoffatomen geknüpft ist.

8. Verfahren gemäss einem der Ansprüche 4 bis 6, dadurch gekennzeichnet, dass man von einem Produkt der Formel (III) ausgeht, worin m = 1 oder 2 und $R_3$ einen Phenyl- oder Pyridinylrest bedeutet.

**Claims for the Contracting States:**
BE, CH, DE, FR, GB, IT, LI, LU, NL, SE

1. Compounds with the formula (I):

$$R_1S\text{-}(CH_2)_n\text{-}\underset{\displaystyle R_2}{\underset{|}{CH}}\text{-}\underset{\displaystyle O}{\underset{\|}{C}}\text{-}NH\text{-}(CH_2)_m\text{-}R_3 \qquad (I)$$

in which $R_1$ represents a hydrogen atom or a $-\underset{\displaystyle O}{\underset{\|}{C}}-R'_1$ radical, $R'_1$ being an alkyl radical containing from 1 to 5 carbon atoms or an aryl radical possibly substituted by a hydroxy radical, an alkyl radical containing from 1 to 5 carbon atoms, an alkoxy radical containing from 1 to 5 carbon atoms, the nitro radical or a halogen atom, n represents a whole number variable from 1 to 5, $R_2$ represents a hydrogen atom, an alkyl radical containing from 1 to 5 carbon atoms, an aryl or arylalkyl radical containing from 6 to 15 carbon atoms, possibly substituted by an alkyl radical containing from 1 to 5 carbon atoms, an alkoxy radical containing from 1 to 5 carbon atoms, a hydroxyl radical, a halogen atom or a trifluoromethyl radical, m represents a whole number variable from 0 to 5, $R_3$ represents:

— either a saturated cyclic radical containing from 3 to 12 carbon atoms, which can be fused to a phenyl radical, $R_3$ however not being able to represent a cyclohexyl radical when $R_2$ and $R_1$ represent at the same time a hydrogen atom and n = 1 and m = 0;

— or a phenyl radical possibly substituted by one or more radicals chosen from the following radicals:- alkyl containing 1 to 5 carbon atoms, alkoxy containing from 1 to 5 carbon atoms, hydroxyl, nitro, halogeno, trifluoromethyl and $-N\begin{smallmatrix}X\\X'\end{smallmatrix}$, X and X' representing a hydrogen atom or an alkyl radical containing from 1 to 5 carbon atoms or a phenyl radical to which a saturated cyclic radical containing from 5 to 9 carbon atoms is fused;

— or a heterocyclic radical chosen from the following radicals:- thiazolyl, 4-5-dihydrothiazolyl, pyridinyl, oxazolyl, isoxazolyl, imidazolyl, pyrimidyl, tetrazolyl, benzimidazolyl, benzothiazolyl, benzoxazolyl, possibly substituted by an alkyl radical containing from 1 to 5 carbon atoms, or a piperidinyl radical substituted by an aralkyl radical containing from 7 to 15 carbon atoms, provided that when $R_3$ represents a phenyl radical possibly substituted by at least one of the radicals previously quoted or when $R_3$ represents one of the heterocyclic radicals mentioned above, possibly substituted by an alkyl radical containing from 1 to 5 carbon atoms, m cannot represent the value 0 and provided that:

a) when n = 1 or 2, m = 1, 2 or 3 and $R_1 = R_2 = H$, $R_3$ is not a phenyl radical possibly substituted by one or two alkyl radicals containing from 1 to 5 carbon atoms,

b) when m = n = 1 and $R_1 = CH_3\text{-}\underset{\displaystyle O}{\underset{\|}{C}}\text{-}$ and $R_2 = CH_3$ $R_3$ is not a phenyl radical, as well as their addition salts with acids.

2. Compounds with the formula (I), as defined in claim 1, characterized in that $R_1$ is a hydrogen atom.

3. Compounds with the formula (I), as defined in claim 1, characterized in that $R_1$ is an acetyl radical.

4. Compounds with the formula (I), as defined in any one of the claims 1 to 3, characterized in that n = 1 and $R_2$ is a benzyl radical or a hydrogen atom.

5. Compounds with the formula (I), as defined in any of the claims 1 to 4, characterized in that m = 0, 1 or 2 and $R_3$ represents a saturated cyclic radical with 3 to 8 carbon atoms possibly fused to a phenyl radical or $R_3$ represents a phenyl radical, to which a saturated cyclic radical containing from 5 to 9 carbon atoms is fused.

6. Compounds with the formula (I) as defined in any one of the claims 1 to 4, characterized in that m = 1 or 2 and $R_3$ represents a phenyl or pyridinyl radical.

7. Compounds with the formula (I), as defined in any of the claims 1 to 6, of which the names follow:
— N-cyclohexyl-α-(mercaptomethyl) benzene propanamide,
— α-(mercaptomethyl)-N-(2-phenylethyl) benzene propanamide,
— N-(2,3-dihydro-1H-inden-2-yl)-3-mercapto propanamide,
— α-(mercaptomethyl)-N-(2-pyridinylmethyl) benzene propanamide,
— α-(mercaptomethyl)-N-(3-pyridinylmethyl) benzene propanamide,
— α-(mercaptomethyl)-N-(4-pyridinylmethyl) benzene propanamide,
as well as their addition salts with acids.

8. Preparation process for products with the formula (I), in which $R_1$, $R_2$, $R_3$ and m have the significances given in claim 1 and in which n = 1, characterized in that an acid with the formula (II):

$$R'_1\text{-}\overset{\displaystyle O}{\overset{\|}{C}}\text{-}S\text{-}CH_2\text{-}\underset{\displaystyle R_2}{\underset{|}{CH}}\text{-}COOH \qquad (II)$$

in which $R'_1$ and $R_2$ have the significances already indicated, or a functional derivative of this acid, is submitted to the action of a product with the formula (III):

$$H_2N\text{-}(CH_2)_m\text{-}R_3 \qquad \text{(III)}$$

in which $R_3$ and m have the significances already indicated, so as to obtain a product with the formula (I), in which $R_1 = R'_1\text{-C-}$, $R'_1$ as well as $R_2$, $R_3$ and m hav-

||
O

ing the significances already indicated and in which $n = 1$, which products with the formula (I) are saponified, if necessary or desired, so as to obtain a product with the formula (I) in which $R_1$ represents a hydrogen atom and which products with the formula (I) are salified, if necessary or desired, by the action of an acid.

9. Preparation process for products with the formula (I), in which $R_1$, $R_2$, $R_3$ and m have the significances given in claim 1 and in which n represents a whole number variable from 1 to 5, characterized in that an acid with the formula (IV):

$R_2$
|
$X\text{-}(CH_2)_n\text{-}CH\text{-}COOH$ (IV)

in which X is a halogen atom, and in which n and $R_2$ have the significances given above, or a functional derivative of this acid, is submitted to the action of a product with the formula (III) $H_2N(CH_2)_mR_3$ as defined in claim 8, so as to obtain a product with the formula (V):

$R_2$
|
$X\text{-}(CH_2)_n\text{-}CH\text{-}COHN\text{-}(CH_2)_m\text{-}R_3$ (V)

in which X, n, m, $R_2$ and $R_3$ have the previous significances, with is submitted to the action of the anion of a thioacid with the formula (VI):

$R'_1\text{-C-SH}$ (VI)
||
O

in which $R'_1$ is defined as in claim 8, so as to obtain a product with the formula (I) in which $R_1 = R'_1\text{-C-}$,

||
O

$R'_1$, $R_2$, $R_3$, n and m having the significances given above, which product with the formula (I) is saponified, if necessary or desired, so as to obtain a product with the formula (I) in which $R_1$ represents a hydrogen atom and which products with the formula (I) are salified, if necessary or desired, by the action of an acid.

10. As medicaments, products with the formula (I), as defined in any one of the claims 1 to 6, as well as their addition salts with pharmaceutically acceptable acids.

11. As medicaments, products as defined in claim 7, as well as their addition salts with pharmaceutically acceptable acids.

12. Pharmaceutical compositions containing as active principle at least one of the medicaments as defined in claims 10 and 11.

**Claims for the Contracting State:** AT

1. Process for preparing compounds with the formula (I):

$R_1S\text{-}(CH_2)_n\text{-}CH\text{-}C\text{-}NH\text{-}(CH_2)_m\text{-}R_3$ (I)
| ||
$R_2$ O

in which $R_1$ represents a hydrogen atom or a $\text{-C-}R'_1$

||
O

radical, $R'_1$ being an alkyl radical containing from 1 to 5 carbon atoms or an aryl radical possibly substituted by a hydroxy radical, an alkyl radical containing from 1 to 5 carbon atoms, an alkoxy radical containing from 1 to 5 carbon atoms, a nitro radical or a halogen atom, n represents a whole number variable from 1 to 5, $R_2$ represents a hydrogen atom, an alkyl radical containing from 1 to 5 carbon atoms, an aryl or arylalkyl radical containing from 6 to 15 carbon atoms, possibly substituted by an alkyl radical containing from 1 to 5 carbon atoms, an alkoxy radical containing from 1 to 5 carbon atoms, a hydroxyl radical, a halogen atom or a trifluoromethyl radical, m represents a whole number variable from 0 to 5, $R_3$ represents:

— either a saturated cyclic radical containing from 3 to 12 carbon atoms, which can be fused to a phenyl radical, $R_3$ howerer not being able to represent a cyclohexyl radical when $R_2$ and $R_1$ represent at the same time a hydrogen atom and $n = 1$ and $m = 0$;

— or a phenyl radical possibly substituted by one or more radicals chosen from the following radicals: alkyl containing 1 to carbon atoms, alkoxy containing from 1 to 5 carbon atoms, hydroxyl, nitro, halogeno, trifluoromethyl and $\text{-N}\langle^{X}_{X'}$, X and X' representing a hydrogen atom or an alkyl radical containing from 1 to 5 carbon atoms or a phenyl radical to which a saturated cyclic radical containing from 5 to 9 carbon atoms is fused;

— or a heterocyclic radical chosen from the following radicals: thiazolyl, 4-5-dihydrothiazolyl, pyridinyl, oxazolyl, isoxazolyl, imidazolyl, pyrimidyl, tetrazolyl, benzimidazolyl, benzothiazolyl, benzoxazolyl, possibly substituted by an alkyl radical containing from 1 to 5 carbon atoms, or a piperidinyl radical substituted by an aralkyl radical containing from 7 to 15 carbon atoms, provided that when $R_3$ represents a phenyl radical possibly substituted by at least one of the radicals previously quoted or when $R_3$ represents one of the heterocyclic radicals mentioned above, possibly substituted by an alkyl radical containing from 1 to 5 carbon atoms, m cannot represent the value 0 and provided that:

a) when $n = 1$ or 2, $m = 1$, 2 or 3 and $R_1 = R_2 = H$, $R_3$ is not a phenyl radical possibly substituted by

one or two alkyl radicals containing from 1 to 5 carbon atoms,

b) when m = n = 1 and $R_1 = CH_3\text{-}C\text{-}$ and $R_2 = CH_3$

$$\begin{array}{c} \| \\ O \end{array}$$

$R_3$ is not a phenyl radical, as well as their addition salts with acids, characterized in that

— to prepare compounds with the formula (I) in which $R_1$, $R_2$ and $R_3$ and m have the previously given significance and in which n = 1 as well as their addition salts with acids, an acid with the formula (II):

$$\begin{array}{l} \quad\; O \\ \quad\; \| \\ R'_1\text{-C-S-}CH_2\text{-CH-COOH} \\ \qquad\qquad\quad\;\; | \\ \qquad\qquad\quad\;\; R_2 \end{array} \qquad (II)$$

in which $R'_1$ and $R_2$ have the significances already indicated, or a functional derivative of this acid, is submitted to the action of a product with the formula (III):

$$H_2N\text{-}(CH_2)_m\text{-}R_3 \qquad (III)$$

in which $R_3$ and m have the significances already indicated, so as to obtain a product with the formula (I), in which $R_1 = R'_1\text{-C-}$, $R'_1$ as well as $R_2$, $R_3$ and m

$$\begin{array}{c} \| \\ O \end{array}$$

having the significances already indicated and in which n = 1, which products with the formula (I) are saponified, if necessary or desired, so as to obtain a product with the formula (I) in which $R_1$ represents a hydrogen atom and which products with the formula (I) are salified, if necessary or desired, by the action of an acid in order to form the salts,

— to prepare the compounds with the formula (I) in which $R_1$, $R_2$ and $R_3$ and m have the previously given significance and in which n represents a whole number variable from 1 to 3, an acid with the formula (IV):

$$\begin{array}{l} \qquad\qquad R_2 \\ \qquad\qquad | \\ X\text{-}(CH_2)_n\text{-CH-COOH} \end{array} \qquad (IV)$$

in which X is a halogen atom, and in which n and $R_2$ have the significances given above, or a functional derivative of this acid, is submitted to the action of a product with the formula (III) $H_2N(CH_2)_mR_3$ as defined previously, so as to obtain a product with the formula (V):

$$\begin{array}{l} \qquad\qquad R_2 \\ \qquad\qquad | \\ X\text{-}(CH_2)_n\text{-CH-COHN-}(CH_2)_m\text{-}R_3 \end{array} \qquad (V)$$

in which X, n, m, $R_2$ and $R_3$ have the previous significances, which is submitted to the action of the anion of a thioacid with the formula (VI):

$$\begin{array}{l} R'_1\text{-C-SH} \\ \quad\;\; \| \\ \quad\;\; O \end{array} \qquad (VI)$$

in which $R'_1$ is defined as previously, so as to obtain a product with the formula (I) in which $R_1 = R'_1\text{-C-}$,

$$\begin{array}{c} \| \\ O \end{array}$$

$R'_1$, $R_2$, $R_3$ n and m having the significances given above, which product with the formula (I) is saponified, if necessary or desired, so as to obtain a product with the formula (I) in which $R_1$ represents a hydrogen atom and which products with the formula (I) are salified, if necessary or desired, by the action of an acid so as to form the salts.

2. Process according to claim 1, for preparing compounds with the formula (I) in which $R_1$, $R_2$ and $R_3$ and n have the previously given significances and in which n = 1, as well as their addition salts with acids, characterized in that an acid with the formula (II):

$$\begin{array}{l} \quad\; O \\ \quad\; \| \\ R'_1\text{-C-S-}CH_2\text{-CH-COOH} \\ \qquad\qquad\quad\;\; | \\ \qquad\qquad\quad\;\; R_2 \end{array} \qquad (II)$$

in which $R'_1$ and $R_2$ have the significances already indicated, or a functional derivative of this acid, is submitted to the action of a product with the formula (III):

$$H_2N\text{-}(CH_2)_m\text{-}R_3 \qquad (III)$$

in which $R_3$ and m have the significances already indicated, so as to obtain a product with the formula (I), in which $R_1 = R'_1\text{-C-}$, $R'_1$ as well as $R_2$, $R_3$ and m

$$\begin{array}{c} \| \\ O \end{array}$$

having the previously given significances and in which n = 1, which products with the formula (I) are saponified, if necessary or desired, so as to obtain a product with the formula (I) in which $R_1$ represents a hydrogen atom and which products with the formula (I) are salified, if necessary or desired, by the action of an acid so as to form the salts.

3. Process according to claim 1, for preparing compounds with the formula (I), in which $R_1$, $R_2$ and $R_3$ and m have the previously given significances and in which n represents a whole number variable from 1 to 3, characterized in that an acid with the formula (IV):

$$\begin{array}{l} \qquad\qquad R_2 \\ \qquad\qquad | \\ X\text{-}(CH_2)_n\text{-CH-COOH} \end{array} \qquad (IV)$$

in which X is a halogen atom, and in which n and $R_2$ have the significances given above, or a functional derivative of this acid, is submitted to the action of a product with the formula (III) $H_2N(CH_2)_mR_3$ as defined previously, so as to obtain a product with the formula (V):

$$\begin{array}{l} \qquad\qquad R_2 \\ \qquad\qquad | \\ X\text{-}(CH_2)_n\text{-CH-COHN-}(CH_2)_m\text{-}R_3 \end{array} \qquad (V)$$

in which X, n, m, $R_2$ and $R_3$ have the previous sig-

nificances, which is submitted to the action of the anion of a thioacid with the formula (VI):

$$R'_1\text{-}\underset{\underset{O}{\|}}{C}\text{-}SH \quad (VI)$$

in which $R'_1$ is defined as previously, so as to obtain a product with the formula (I) in which $R_1 = R'_1\text{-}\underset{\underset{O}{\|}}{C}\text{-}$, $R'_1$, $R_2$, $R_3$, n and m having the significances given above, which product with the formula (I) is saponified, if necessary or desired, so as to obtain a product with the formula (I) in which $R_1$ represents a hydrogen atom and which products with the formula (I) are salified, if necessary or desired, by the action of an acid so as to form the salts.

4. Process according to claim 1, for the preparation of compounds with the formula (I) as defined in claim 1, answering to the formula (I'):

$$R_1\text{-}S\text{-}(CH_2)_n\text{-}\underset{\underset{R_2}{|}}{CH}\text{-}\underset{\underset{O}{\|}}{C}\text{-}NH\text{-}(CH_2)_m\text{-}R'_3 \quad (I)$$

in which $R_1$, $R_2$, n and m keep their previously given significances and $R'_3$ represents

— either a saturated cyclic radical containing from 3 to 12 carbon atoms, which can be fused to a phenyl radical, $R_3$ howerer not being able to represent a cyclohexyl radical when $R_2$ and $R_1$ represent at the same time a hydrogen atom, and $n = 1$ and $m = 0$;

— or a phenyl radical possibly substituted by one or more radicals chosen from the following radicals: alkyl containing 1 to 5 carbon atoms, alkoxy containing from 1 to 5 carbon atoms, hydroxyl, nitro, halogeno, trifluormethyl and $-N\genfrac{}{}{0pt}{}{X}{X'}$, X and X' representing a hydrogen atom or an alkyl radical containing from 1 to 5 carbon atoms or a phenyl radical to which a saturated cyclic radical containing from 5 to 9 carbon atoms is fused;

— or a heterocyclic radical chosen from the following radicals: thiazolyl, 4-5-dihydrothiazolyl, pyridinyl, oxazolyl, isoxyzolyl, imidazolyl, pyrimidyl, tetrazolyl, benzimidazolyl, benzothiazolyl, benzoxazolyl, possibly substituted by an alkyl radical containing from 1 to 5 carbon atoms, or a piperidinyl radical substituted by an aralkyl radical containing from 7 to 15 carbon atoms, provided that when $R'_3$ represents a phenyl radical possibly substituted by at least one of the radicals previously quoted or when $R'_3$ represents one of the heterocyclic radicals mentioned above, possibly substituted by an alkyl radical containing from 1 to 5 carbon atoms, m cannot represent the value 0 and provided that:

a) when $n = 1$ or 2, $m = 1$, 2 or 3 and $R_1 = R_2 = H$, $R'_3$ is not a phenyl radical possibly substituted by one or two alkyl radicals containing from 1 to 5 carbon atoms,

b) when $m = n = 1$ and $R_1 = CH_3\text{-}\underset{\underset{O}{\|}}{C}\text{-}$ and $R_2 = CH_3$ $R'_3$ is not a phenyl radical, as well as their addition salts with acids, characterized in that at the start a product with the formula (III) is used, in which $R_3$ has the values for $R'_3$ indicated above.

5. Process according to claim 4, characterized in that at the start a product with the formula (II) or (VI) is used, in which $R'_1\text{-}\underset{\underset{O}{\|}}{C}\text{-}$ represents an acetyl radical.

6. Process acording to claim 5, characterized in that at the start a product with the formula (V) or (IV) is used, in which $R_2$ represents a benzyl radical or a hydrogen atom and n is equal to 1.

7. Process according to any of the claims 4 to 6, characterized in that at the start a product with the formula (III) is used, in which $m = 0$, 1 or 2, $R_3$ represents a saturated cyclic radical with 3 to 8 carbon atoms, possibly fused to a phenyl radical or $R_3$ represents a phenyl radical to which a saturated cyclic radical containing from 5 to 9 carbon atoms is fused.

8. Process according to any one of the claims 4 to 6, characterized in that at the start a product with the formula (III) is used, in which $m = 1$ or 2 and $R_3$ represents a phenyl or pyridinyl radical.